# EUROPEAN PATENT SPECIFICATION

(11) **EP 1 254 956 B1**
(45) Date of publication and mention of the grant of the patent: **31.12.2008**
(21) Application number: 02014841.7
(22) Date of filing: 30.06.1987
(51) Int. Cl.: C12N 15/12, C07K 14/51, A61K 38/17

(54) **Novel osteoinductive compositions**
Osteoinduktive Zusammensetzungen
Compositions ostéoinductrices

(30) Priority: 01.07.1986 US 880776; 17.12.1986 US 943332; 20.03.1987 US 28285; 26.03.1987 US 31346
(43) Date of publication of application: 06.11.2002
(62) Divisional of application: 95111771.2
(73) Proprietor: Genetics Institute, LLC, Cambridge, MA 02140 (US)
(72) Inventor: Wang, Elizabeth A., Carlisle, MA 01741 (US); Wozney, John M., Hudson, MA 01749 (US); Rosen, Vicki A., Chestnut Hill, MA 02467 (US)
(74) Representative: Denholm, Anna Marie

(56) References cited:
- EP-A- 0 121 976
- EP-A- 0 128 041
- EP-A- 0 148 155
- EP-A- 0 212 474
- WO-A-85/04173
- US-A- 4 455 256
- US-A- 4 563 350
- US-A- 4 619 989
- URIST M R ET AL: "BONE CELL DIFFERENTIATION AND GROWTH FACTORS" SCIENCE, AMERICAN ASSOCIATION FOR THE ADVANCEMENT OF SCIENCE, US, vol. 4598, no. 220, 1 May 1983 (1983-05-01), pages 680-686, XP008003037 ISSN: 0036-8075
- URIST M ET AL: "THE BIOLOGICALLY-ACTIVE N-TERMINAL REGION OF THE BONE MORPHOGENETIC PROTEIN (BMP) MOLECULE ISOLATED BY PEPSIN LIMITED PROTEOLYSIS" FEDERATION PROCEEDINGS, BETHESDA, MD, US, vol. 3, no. 44, 1985, page 746 XP008003036 ISSN: 0014-9446
- URIST M R ET AL: "Purification of bovine bone morphogenetic protein by hydroxyapatite chromatography" PROCEEDINGS OF THE NATIONAL ACADEMY OF SCIENCES OF USA, NATIONAL ACADEMY OF SCIENCE. WASHINGTON, US, vol. 81, no. 2, January 1984 (1984-01), pages 371-375, XP002198250 ISSN: 0027-8424
- URIST M R ET AL: "BETA-TRICALCIUM PHOSPHATE DELIVERY SYSTEM FOR BONE MORPHOGENETIC PROTEIN" CLINICAL ORTHOPAEDICS AND RELATED RESEARCH, PHILADELPHIA, PA, US, vol. 187, July 1984 (1984-07), pages 277-280, XP008003034
- URIST M R ET AL: "HUMAN BONE MORPHOGENETIC PROTEIN (HBMP) (41630)" PROCEEDINGS OF THE SOCIETY FOR EXPERIMENTAL BIOLOGY & MEDICINE, WILLIAMS AND WILKINS, XX, vol. 2, no. 173, June 1983 (1983-06), pages 194-199, XP008003032 ISSN: 0037-9727
- SAMPATH T K ET AL: "HOMOLOGY OF BONE INDUCTIVE PROTEINS FROM HUMAN MONKEY BOVINE AND RAT EXTRACELLULAR MATRIX" PROCEEDINGS OF THE NATIONAL ACADEMY OF SCIENCES OF THE UNITED STATES, vol. 80, no. 21, 1983, pages 6591-6595, XP002213538 1983 ISSN: 0027-8424

## Description

The present invention relates to novel proteins and processes for obtaining them. These proteins are capable of inducing cartilage and bone formation.

### Background

Bone is a highly specialized tissue characterized by an extensive matrix structure formed of fibrous bundles of the protein collagen, and proteoglycans, noncollagenous proteins, lipids and acidic proteins. The processes of bone formation and renewal/repair of bone tissue, which occur continuously throughout life, are performed by specialized cells. Normal embryonic long bone development is preceded by formation of a Cartilage model. Bone growth is presumably mediated by "osteoblasts" (bone-forming cells), while remodeling of bone is apparently accomplished by the joint activities of bone-resorbing cells, called "osteoclasts" and osteoblasts. A variety of osteogenic, cartilage-inducing and bone inducing factors have been described. See, e.g. European patent applications 148,155 and 169,016 for discussions thereof.

### Brief Description of the Invention

The present invention provides a novel protein in purified form BMP-1 wherein BMP is bone morphogenic protein. This protein is characterized by peptide sequences the same as or substantially homologous to amino acid sequences illustrated in Tables V and VI below. It is capable of inducing bone formation at a predetermined site. This bone inductive factor is further characterized by biochemical and biological characteristics including activity at a concentration of 10 to 1000ng/gram of bone in an in vivo rat bone formation assay described below. Proteins of this invention may be encoded by the DNA sequences depicted in the Tables or by sequences capable of hybridizing thereto under stringent conditions and coding for polypeptides with bone growth factor biological properties or other variously modified sequences demonstrating such properties.

One of the proteins of the invention is designated BMP-1. A portion of the human BMP-1 or hBMP-1 is characterized by the same or substantially the same peptide sequence as that of amino acid #1 through amino acid #37 of Table V, below which represents a genomic hBMP-1 fragment or amino acid #1 through amino acid #730 of Table VI which represents the hBMP-1 cDNA. hBMP-1 or a related bone inductive factor may be further characterized by at least a portion of these sequences. These peptide sequences are encoded by the same or substantially the same DNA sequence, as depicted in nucleotide #3440 through nucleotide #3550 of Table V and in nucleotide #36 through nucleotide #2225 of Table VI, respectively. These hBMP-1 polypeptides are further characterized by the ability to induce bone formation. hBMP-1 demonstrates activity in an in vivo rat bone formation assay at a concentration of 10 to 1000ng/gram of bone.

The homologous bovine growth factor of the invention, designated bBMP-1, is characterized by a peptide sequence containing the same or substantially the same sequence as that of amino acid #1 through amino acid #37 of Table II below which represents a genomic bBMP-1 fragment. This peptide sequence is encoded by the same or substantially the same DNA sequence as depicted in nucleotide #294 through nucleotide #404 of Table II. The bovine peptide sequence identified in Table II below is also 37 amino acids in length. bBMP-1 is further characterized by the ability to induce bone formation.

Another aspect of the invention provides pharmaceutical compositions containing a therapeutically effective amount of the bone growth factor polypeptide according to the invention in a pharmaceutically acceptable vehicle. These compositions may further include other therapeutically useful agents. They may also include an appropriate matrix for delivering the proteins to the site of the bone defect and for providing a structure for bone growth. These compositions may be employed in methods for treating a number of bone defects and periodontal disease. These methods, according to the invention, entail administering to a patient needing such bone formation an effective amount of the novel protein BMP-1, as described herein.

Still a further aspect of the invention are DNA sequences coding on expression for a human or bovine polypeptide having the ability to induce bone formation. Such sequences include the sequence of nucleotides in a 5' to 3' direction illustrated in Tables V and VI. Alternatively, a DNA sequence which hybridizes under stringent conditions with the DNA sequences of Tables V and VI and which codes on expression for a protein having at least one bone growth factor biological property are included in the present invention. Finally, allelic or other variations of the sequences of Tables V and VI, whether such nucleotide changes result in changes in the peptide sequence or not, are also included in the present invention.

Still a further aspect of the invention is a vector containing a DNA sequence as described above in operative association with an expression control sequence. Such vector may be employed in a novel process for producing a bone growth factor polypeptide in which a cell line transformed with a DNA sequence encoding expression of a bone growth factor polypeptide in operative association with an expression control sequence therefor, is cultured. This claimed process may employ a number of known cells as host cells for expression of the polypeptide. Presently preferred cell lines are mammalian cell lines and bacterial cells.

Other aspects and advantages of the present invention will be apparent upon consideration of the following detailed description and preferred embodiments thereof.

### Detailed Description of the Invention

The protein of the present invention is characterized by amino acid sequences or portions thereof the same as or substantially homologous to the sequences shown in Tables V and VI below. This protein is also characterized by the ability to induce bone formation.

The bone growth factors provided herein also include factors encoded by the sequences similar to those of Tables V and VI, but into which modifications are naturally provided (e.g. allelic variations in the nucleotide sequence which may result in amino acid changes in the polypeptide) or deliberately engineered. For example, synthetic polypeptides may wholly or partially duplicate continuous sequences of the amino acid residues of Tables V and VI. These sequences, by virtue of sharing primary, secondary, or tertiary structural and conformational characteristics with the bone growth factor polypeptides of Tables V and VI may possess bone growth factor biological properties in common therewith. Thus, they may be employed as biologically active substitutes for naturally-occurring bone growth factor polypeptides in therapeutic processes.

Other specific mutations of the sequences of the bone growth factor described herein involve modifications of one or both of the glycosylation sites. The absence of glycosylation or only partial glycosylation results from amino acid substitution or deletion at one or both of the asparagine-linked glycosylation recognition sites present in the sequences of the bone growth factor shown in Tables V and VI. The asparagine-linked glycosylation recognition sites comprise tripeptide sequences which are specifically recognized by appropriate cellular glycosylation enzymes. These tripeptide sequences are either asparagine-X-threonine or asparagine-X-serine, where X is usually any amino acid. A variety of amino acid substitutions or deletions at one or both of the-first or third amino acid positions of a glycosylation recognition site (and/or amino acid deletion at the second position) results in non-glycosylation at the modified tripeptide sequence.

The present invention also encompasses the novel DNA sequences, free of association with DNA sequences encoding other proteinaceous materials, and coding on expression for bone growth factors. These DNA sequences include those depicted in Tables V and VI in a 5' to 3' direction and those sequences which hybridize under stringent hybridization conditions [see, T. Maniatis et al, Molecular Cloning (A Laboratory Manual), Cold Spring Harbor Laboratory (1982), pages 387 to 389 to the DNA sequences of Tables V and VI.

Similarly, DNA sequences which code for bone growth factor polypeptides coded for by the sequences of Tables V and VI, but which differ in codon sequence due to the degeneracies of the genetic code or allelic variations (naturally-occurring base changes in the species population which may or may not result in an amino acid change) also encode the novel growth factors described herein. Variations in the DNA sequences of Tables V and VI which are caused by point mutations or by induced modifications to enhance the activity, half-life or production of the polypeptides encoded thereby are also encompassed in the invention.

Another aspect of the present invention provides a novel method for producing the novel osteoinductive factors. The method of the present invention involves culturing a suitable cell or cell line, which has been transformed with a DNA sequence coding on expression for a novel bone growth factor polypeptide of the invention, under the control of known regulatory sequences. Suitable cells or cell lines may be mammalian cells, such as Chinese hamster ovary cells (CHO). The selection of suitable mammalian host cells and methods for transformation, culture, amplification, screening and product production and purification are known in the art. See, e.g., Gething and Sambrook, Nature, 293:620-625 (1981), or alternatively, Kaufman et al, Mol. Cell. Biol., 5(7):1750-1759 (1985) or Howley et al, U.S. Patent 4,419,446. Another suitable mammalian cell line, which is described in the accompanying examples, is the monkey COS-1 cell line. A similarly useful mammalian cell line is the CV-1 cell line.

Bacterial cells are suitable hosts. For example, the various strains of E. coli (e.g., HB101, MC1061) are well-known as host cells in the field of biotechnology. Various strains of B. subtilis, Pseudomonas, other bacilli and the like may also be employed in this method.

Many strains of yeast cells known to those skilled in the art are also available as host cells for expression of the polypeptides of the present invention. Additionally, where desired, insect cells may be utilized as host cells in the method of the present invention. See, e.g. Miller et al, Genetic Engineering, 8:277-298 (Plenum Press 1986) and references cited therein.

Another aspect of the present invention provides vectors for use in the method of expression of these novel osteoinductive polypeptides. Preferably the vectors contain the full novel DNA sequences described above which code for the novel factors of the invention. Additionally the vectors also contain appropriate expression control sequences permitting expression of the bone inductive protein sequences. Alternatively, vectors incorporating modified sequences as described above are also embodiments of the present invention and useful in the production of the bone inductive proteins. The vectors may be employed in the method of transforming cell lines and contain selected regulatory sequences in operative association with the DNA coding sequences of the invention which are capable of directing the replication and expression thereof in selected host cells. Useful regulatory sequences for such vectors are known to one of skill in the art and may be selected depending upon the selected host cells. Such selection is routine and does not form part of the present invention.

A protein of the present invention, which induces bone growth in circumstances where bone is not normally formed, has application in the healing of bone fractures. An osteogenic preparation employing a protein of the invention may have prophylactic use in closed as well as open fracture reduction and also in the improved fixation of artificial joints. De novo bone formation induced by an osteogenic agent contributes to the repair of congenital, trauma induced, or oncologic resection induced craniofacial defects, and also is useful in cosmetic plastic surgery. An osteogenic factor of the invention may be valuable in the treatment of periodontal disease, and in other tooth repair processes. Such agents may provide an environment to attract bone-forming cells, stimulate growth of bone-forming cells or induce differentiation of progenitors of bone-forming cells. Of course, the proteins of the invention may have other therapeutic use.

A further aspect of the invention is a therapeutic method and composition for repairing fractures and other conditions related to bone defects or periodontal diseases. Such a composition comprises a therapeutically effective amount of the bone inductive factor protein of the invention. The bone inductive factor according to the present invention may be present in a therapeutic composition in admixture with a pharmaceutically acceptable vehicle or matrix. Further therapeutic methods and compositions of the invention comprise a therapeutic amount of a bone inductive factor of the invention with a therapeutic amount of at least one of the other bone inductive factors of the invention. Additionally, the protein according to the present invention may be co-administered with one or more different osteoinductive factors with which it may interact. Further, the bone inductive protein may be combined with other agents beneficial to the treatment of the bone defect in question. Such agents include, but are not limited to various growth factors. The preparation of such physiologically acceptable protein compositions, having due regard to pH, isotonicity, stability and the like, is within the skill of the art.

In particular, BMP-1 may be used individually in a composition. BMP-1 may also be used in combination with one or more of the other proteins. BMP-1 and BMP-2 Class I may be used in combination. BMP-1 and BMP-2 Class II may also be used in combination. BMP-1 and BMP-3 may be used in combination. Furthermore, BMP-1 may be used in combination with two or three of the other proteins of the invention. For example, BMP-1, BMP-2 Class I, and BMP-2 Class II may be combined. BMP-1 may also be combined with BMP-2 Class I, and BMP-3. Further, BMP-1 may be combined with BMP-2 Class II, and BMP-3. BMP-1, BMP-2 Class I, BMP-2 Class -II, and BMP-3 may be combined.

The therapeutic method includes locally administering the composition as an implant or device. When administered, the therapeutic composition for use in this invention is, of course, in a pyrogen-free, physiologically acceptable form. Further, the composition may desirably be encapsulated or injected in a viscous form for delivery to the site of bone damage. Preferably, the bone growth inductive factor composition would include a matrix capable of delivering the bone inductive factor to the site of bone damage, providing a structure for the developing bone and cartilage and optimally capable of being resorbed into the body. Such matrices may be formed of other materials presently in use for other implanted medical applications.

The choice of material is based on, for example, biocompatibility, biodegradability, mechanical properties, cosmetic appearance and interface properties. Similarly, the application of the osteoinductive factors will define the appropriate formulation. Potential matrices for the osteoinductive factors may be biodegradable and chemically defined, such as, but not limited to calcium sulfate, tricalciumphosphate, hydroxyapatite, polylactic acid, polyanhydrides; biodegradable and biologically well defined, such as bone or dermal collagen, other pure proteins or extracellular matrix components; nonbiodegradable and chemically defined, such as sintered hydroxyapatite, bioglass, aluminates, or other ceramics; or combinations of any of the above mentioned types of material, such as polylactic acid and hydroxyapatite or collagen and tricalciumphosphate. The bioceramics might also be altered in composition, such as in calcium-aluminate-phosphate and processing to alter for example, pore size, particle size, particle shape, and biodegradability.

The dosage regimen will be determined by the attending physician considering various factors which modify the action of such a growth factor, e.g. amount of bone weight desired to be formed, the site of bone damage, the condition of the damaged bone, the patient's age, sex, and diet, the severity of any infection, time of administration and other clinical factors. The dosage may vary with the type of matrix used in the reconstitution and the composition of BMF's. The addition of other known growth factors, such as IGF 1 (insulin like growth factor 1), to the final composition, may also effect the dosage. Generally, the dosage regimen should be in then range of approximately 10 to 10⁶ nanograms of protein per gram of bone weight desired. Progress can be monitored by periodic assessment of bone growth and/or repair, e.g. x-rays. Such therapeutic compositions are also presently valuable for veterinary applications due to the lack of species specificity in bone inductive factors. Particularly domestic animals and thoroughbred horses in addition to humans are desired patients for such treatment with the bone inductive factors of the present invention.

The following examples illustrate practice of the present invention in recovering and characterizing the bovine proteins and employing them to recover the human proteins, obtaining the human proteins and in expressing the proteins via recombinant techniques.

### EXAMPLE I

### Isolation of Bovine Bone Inductive Factor

Ground bovine bone powder (20-120 mesh, Helitrex) is prepared according to the procedures of M. R. Urist et al., Proc. Natl Acad. Sci. USA, 70:3511 (1973) with elimination of some extraction steps as identified below. Ten kgs of the - ground powder is demineralized in successive changes of 0.6N HCl at 4°C over a 48 hour period with vigorous stirring. The resulting suspension is extracted for 16 hours at 4°C with 50 liters of 2M CaCl₂ and 10mM ethylenediamine-tetraacetic acid [EDTA], and followed by extraction for 4 hours in 50 liters of 0.5M EDTA. The residue is washed three times with distilled water before its resuspension in 20 liters of 4M guanidine hydrochloride [GuCl], 20mM Tris (pH 7.4), 1mM N-ethylmaleimide, 1mM iodoacetamide, 1mM phenylmethylsulfonyl fluorine as described in Clin. Orthop. Rel. Res., 171: 213 (1982). After 16 to 20 hours the supernatant is removed and replaced with another 10 liters of GuCl buffer. The residue is extracted for another 24 hours.

The crude GuCl extracts are combined, concentrated approximately 20 times on a Pellicon apparatus with a 10,000 molecular weight cut-off membrane, and then dialyzed in 50mM Tris, 0.1M NaCl, 6M urea (pH7.2), the starting buffer for the first column. After extensive dialysis the protein is loaded on a 4 liter DEAE cellulose column and the unbound fractions are collected.

The unbound fractions are concentrated and dialyzed against 50mM NaAc, 50mM NaCl (pH 4.6) in 6M urea. The unbound fractions are applied to a carboxymethyl cellulose column. Protein not bound to the column is removed by extensive washing with starting buffer, and the bone inductive factor containing material desorbed from the column by 50mM NaAc, 0.25mM NaCl, 6M urea (pH 4.6). The protein from this step elution is concentrated 20- to 40- fold, then diluted 5 times with 80mM KPO₄, 6M urea (pH6.0). The pH of the solution is adjusted to 6.0 with 500mM K₂HPO₄. The sample is applied to an hydroxylapatite column (LKB) equilibrated in 80mM KPO₄, 6M urea ((pH6.0) and all unbound protein is removed by washing the column with the same buffer. Bone inductive factor activity is eluted with 100mM KPO₄ (pH7.4) and 6M urea.

The protein is concentrated approximately 10 times, and solid NaCl added to a final concentration of 0.15M. This material is applied to a heparin - Sepharose column equilibrated in 50mM KPO₄, 150mM NaCl, 6M urea (pH7.4) . After extensive washing of the column with starting buffer, a protein with bone inductive factor activity is eluted by 50mM KPO₄, 700mM NaCl, 6M urea (pH7.4). This fraction is concentrated to a minimum volume, and 0.4ml aliquots are applied to Superose 6 and Superose 12 columns connected in series, equilibrated with 4M GuCl, 20mM Tris (pH7.2) and the columns developed at a flow rate of 0.25ml/min. The protein demonstrating bone inductive factor activity has a relative migration corresponding to approximately 30,000 dalton protein.

The above fractions are pooled, dialyzed against 50mM NaAc, 6M urea (pH4.6), and applied to a Pharmacia MonoS HR column. The column is developed with a gradient to 1.0M NaCl, 50mM NaAc, 6M urea (pH4.6). Active fractions are pooled and brought to (pH3.0 with 10% trifluoroacetic acid (TFA). The material is applied to a 0.46 x 25cm Vydac C4 column in 0.1% TFA and the column developed with a gradient to 90% acetonitrile, 0.1% TFA (31.5% acetonitrile, 0.1% TFA to 49.5% acetonitrile, 0.1% TFA in 60 minutes at 1ml per minute). Active material is eluted at approximately 40-44% acetonitrile. Aliquots of the appropriate fractions are iodinated by one of the following methods: P. J. McConahey et al, Int. Arch. Allergy, 29:185-189 (1966); A. E. Bolton et al, Biochem J., 133:529 (1973); and D. F. Bowen-Pope, J. Biol. Chem., 237:5161 (1982) . The iodinated proteins present in these fractions are analyzed by SDS gel electrophoresis and urea Triton X 100 isoelectric focusing. At this stage, the bone inductive factor is estimated to be approximately 10-50% pure.

### EXAMPLE II

### Characterization of Bovine Bone Inductive Factor

### A. Molecular Weigh

Approximately 20ug protein from Example I is lyophilized and redissolved in 1X SDS sample buffer. After 15 minutes of heating at 37°C, the sample is applied to a 15% SDS polyacrylamide gel and then electrophoresed with cooling. The molecular weight is determined relative to prestained molecular weight standards (Bethesda Research Labs). Immediately after completion, the gel lane containing bone inductive factor is sliced into 0.3cm pieces. Each piece is mashed and 1.4ml of 0 .1% SDS is added. The samples are shaken gently overnight at room temperature to elute the protein. Each gel slice is desalted to prevent interference in the biological assay. The supernatant from each sample is acidified to pH 3.0 with 10% TFA, filtered through a 0.45 micron membrane and loaded on a 0.46cm x 5cm C4 Vydac column developed with a gradient of 0.1% TFA to 0.1% TFA, 90% CH₃CN. The appropriate bone inductive factor - containing fractions are pooled and reconstituted with 20mg rat matrix. In this gel system, the majority of bone inductive factor fractions have the mobility of a protein having a molecular weight of approximately 28,000 - 30,000 daltons.

### B. Isoelectric Focusing

The isoelectric point of bone inductive factor activity is determined in a denaturing isoelectric focusing system. The Triton X100 urea gel system (Hoeffer Scientific) is modified as follows: 1) 40% of the ampholytes used are Servalyte 3/10; 60% are Servalyte 7-9. 2) The catholyte used is 40mM NaOH. Approximately 20ug of protein from. Example I is lyophilized, dissolved in sample buffer and applied to the isoelectrofocusing gel. The gel is run at 20 watts, 10° C for approximately 3 hours. At completion the lane containing bone inductive factor is sliced into 0.5 cm slices. Each piece is mashed in 1.0ml 6M urea, 5mM Tris (pH 7.8) and the samples agitated at room temperature. The samples are acidified, filtered, desalted and assayed as described above. The major portion of activity as determined in the assay described in Example III migrates in a manner consistent with a pI of 8.8 - 9.2.

### C. Subunit Characterization

The subunit composition of bone inductive factor is also determine. Pure bone inductive factor is isolated from a preparative 15% SDS gel as described above. A portion of the sample is then reduced with 5mM DTT in sample buffer and re-electrophoresed on a 15% SDS gel. The approximately 30kd protein yields two major bands at approximately 20kd and 18kd, as well as a minor band at 30kd. The broadness of the two bands indicates heterogeneity caused most probably by glycosylation, other post translational modification, proteolytic degradation or carbamylation.

### EXAMPLE III

### Biological Activity of Bone Inductive Factor

A rat bone formation assay according to the general procedure of Sampath and Reddi, Proc. Natl. Acad. Sci. U.S.A., 80:6591-6595 (1983) is used to evaluate the osteogenic activity of the bovine bone inductive factor of the present invention obtained in Example I. This assay can also be used to evaluate bone inductive factors of other species. The ethanol precipitation step is replaced by dialysing the fraction to be assayed against water. The solution or suspension is then redissolved in a volatile solvent, e.g. 0.1 - 0.2 % TFA, and the resulting solution added to 20mg of rat matrix. This material is frozen and lyophilized and the resulting powder enclosed in #5 gelatin capsules. The capsules are implanted subcutaneously in the abdominal thoracic area of 21 - 49 day old male long Evans rats. The implants are removed after 7-14 days. Half of each implant is used for alkaline phosphatase analysis [See, A. H. Reddi et al., Proc. Natl Acad Sci., 69:1601 (1972)] and half is fixed and processed for histological analysis. Routinely, 1um glycolmethacrylate sections are strained with Von Kossa and acid fuschin to detect new bone mineral. Alkaline phosphatase, an enzyme produced by chondroblasts and osteoblasts in the process of matrix formation, is also measured. New cartilage and bone formation often correlates with alkaline phosphatase levels. Table I below illustrates the dose response of the rat matrix samples including a control not treated with bone inductive factor.

**TABLE 1**

| Protein* Implanted ug | Cartilage | Alk. Phos.u/l |
|---|---|---|
| 7.5 | 2 | Not done |
| 2.5 | 3 | 445.7 |
| 0.83 | 3 | 77.4 |
| 0.28 | 0 | 32.5 |
| 0.00 | 0 | 31.0 |

| | | |
|---|---|---|
| *At this stage the bone inductive factor is approximately 10-15% pure. | | |

The bone or cartilage formed is physically confined to the space occupied by the matrix. Samples are also analyzed by SDS gel electrophoresis and isoelectric focusing as described above, followed by autoradiography. Analysis reveals a correlation of activity with protein bands at 28 - 30kd and a pI 9.0. An extinction coefficient of 1 OD/mg-cm is used as an estimate for protein and approximating the purity of bone inductive factor in a particular fraction. In the in vivo rat bone formation assays on dilutions as described above, the protein is active in vivo at 10 to 200ng protein/gram bone to probably greater than lug protein/gram bone.

### EXAMPLE IV

### Bovine Bone inductive Factor Protein Composition

The protein composition of Example IIA of molecular weight 28 - 30kd is reduced as described in Example IIC and digested with trypsin. Eight tryptic fragments are isolated by standard procedure having the following amino acid sequences:
Fragment 1: A A F L G D I A L D E E D L G
Fragment 2: A F Q V Q Q A A D L
Fragment 3: N Y Q D M V V E G
Fragment 4 : S T P A Q D V S R
Fragment 5: N Q E A L R
Fragment 6: L S E P D P S H T L E E
Fragments 7 : F D A Y Y
Fragment 8 : L-K P S N ? A T I Q S I V E

A less highly purified preparation of protein from bovine bony prepared according to a purification scheme similar to that described in Example I. The purification basically varies from that previously described by omission of the DE-52 column, the CM cellulose column and the mono S column, as well as a reversal in the order of the hydroxylapatite and heparin sepharose columns. Briefly, the concentrted crude 4 M extract is brought to 85% final concentration of ethanol at 4 degrees. The mixture is then centrifuged, and the precipitate redissolved in 50 mM Tris, 0.15 M NaCl, 6.0 M urea. This material is then fractionated on Heparin Sepharose as described. The Heparin bound material is fractionated on hydroxyapatite as described The active fractions are pooled, concentrated, and fractionated on a high resolution gel filtration (TSK 30000 in 6 M guanidinium chloride, 50 mM Tris, pH 7.2). The active fractions are pooled, dialyzed against 0.1% TFA, and then fractionated on a C4 Vydac reverse phase column as described. The preparation is reduced and electrophoresed on an acrylamide gel. The protein corresponding to the 18K band is eluted and digested with trypsin. Tryptic fragments are isolated having the following amino acid sequences:
Fragment 9: S L K P S N H A T I Q S ? V
Fragment 10: S F D A Y Y C S ? A
Fragment 11: V Y P N M T V E S C A
Fragment 12: V D F A D I ? W

Tryptic Fragments 7 and 8 are noted to be substantially the same as Fragments 10 and 9, respectively.

### A. bBMP-1

Probes consisting of pools of oligonucleotides (or unique oligonucleotides) are designed according to the method of R. Lathe, J. Mol. Biol., 183 (1):1-12 (1985) and synthesized on an automated DNA synthesizer. One probe consists of a relatively long (32 nucleotides) "guessmer" [See J. J. Toole et, al, Nature, 312: 3.42-347 (1984)] of the following nucleotide sequence:
TCCTCATCCAGGGCAATGTCGCCCAGGAAGGC

Because the genetic code is degenerate (more than one codon can code for the same amino acid), the number of oligonucleotides in a probe pool is reduced based on the frequency of codon usage in eukaryotes, the relative stability of G:T base pairs, and the relative infrequency of the dinucleotide CpG in eukaryotic coding sequences [see Toole et al., supra.]. The second set of probes consists of shorter oligonucleotides (17 nucleotides in length) which contain all possible sequences that could encode the amino acids. The second set of probes has the following sequences:
(a) A [A/G] [A/G] TC [T/C] TC [T/C] TC [A/G] TC [T/C] AA
(b) A [A/G] [A/G] TC [T/C] TC [T/C] TC [A/G] TCNAG
Bracketed nucleotides are alternatives. "N" means either A, T, C or G.

In both cases the regions of the amino acid sequence used for probes design are chosen by avoiding highly degenerate codons where possible. The oligonucleotides are synthesized on an automated DNA synthesizer; the probes are then radioactively labeled with polynucleotide kinase and ³²P-ATP.

These two sets of probes are used to screen a bovine genomic recombinant library. The library is constructed as follows: Bovine liver DNA is partially digested with then restriction endonuclease enzyme Sau 3A and sedimented through a sucrose gradient. Size fractionated DNA in the range of' 15-30kb is then ligated to the bacteriophage Bam HI vector EMBL3 [Frischauf et al, J. Mol. Biol., 170:827-842 (1983)]. The library is plated at 8000 recombinants per plate. Duplicate nitrocellulose replicas of the plaques are made and amplified according to a modification of the procedure of Woo et al, Proc. Natl. Acad. Sci. USA, 75:3688-91 (1978).

The 32 mer probe is kinased with ³²P-gamma-ATP and hybridized to one set of filters in 5X SSC, 0.1% SDS, 5X Denhardts, 100ug/ml salmon sperm DNA at 45 degrees C and washed with 5X SSC, 0.1% SDS at 45 degrees C. The 17 mer probes are kinased and hybridized to the other set of filters in 3M tetramethylammonium chloride (TMAC) 0.1M sodium phosphate pH6.5, 1mM EDTA, 5X Denhardts, 0.6% SDS, 100ug/ml salmon sperm DNA at 48 degrees C, and washed in 3M TMAC, 50mM Tris pH8.0 at 50 degrees C. these conditions minimize the detection of mismatches to the 17 mer probe pool [see, Wood et al, Proc. Natl. Acad. Sci, U.S.A., 82:1585-1588 (1985)]. 400,000 recombinants are screened by this procedure and one duplicate positive is plaque purified. DNA is isolated from a plate lysate of this recombinant bacteriophage designated lambda bP-50. bP-50 was deposited December 16, 1986 with the American Type Culture Collection, 12301 Parklawn Drive, Rockville, Maryland USA (hereinafter the "ATCC") under accession number 40295. This deposit as well as the other deposits contained herein meets the requirements of the Budapest Treaty on the International Recognition of the Deposit of Microorganisms for the Purposes of Patent Procedure and Regulations thereunder. This bp-50 clone encodes at least a portion of the bovine bone growth factor designated bBMP-1.

The oligonucleotide hybridizing region of this bBMP-1 clone is localized to an approximately 800bp Eco RI fragment which is subcloned into M13 and sequenced by standard techniques. The partial DNA sequence and derived amino acid sequence of lambda bP-50 are shown below in Table II. The amino acid sequences corresponding to the Tryptic fragments isolated from the bovine bone 28 to 30kd material are underlined in Tableau II. The first underlined portion of the sequence corresponds to tryptic Fragment 1 above from which the oligonucleotide probes are designed. The second underlined portion corresponds to Tryptic Fragment 2 above. The predicted amino acid sequence indicates that tryptic Fragment 2 is preceded by a basic residue (R) as expected considering the specificity of trypsin. The nucleic acid sequence preceding the couplet CT at nucleotide positions #292-293 in Table II is presumed to be an intron (noncoding sequence) based on the presence of a consensus acceptor sequence (i.e., a pyrimidine rich tract, TCTCTCTCC, followed by AG) and the lack of a basic residue in the appropriate position of the derived amino acid sequence. This bBMP-1 genomic sequence appears in Table II. The presumptive bBMP-1 peptide sequence from this genomic clone is 37 amino acids in length and is encoded by the DNA sequence from nucleotides #294 through #404 in Table II.

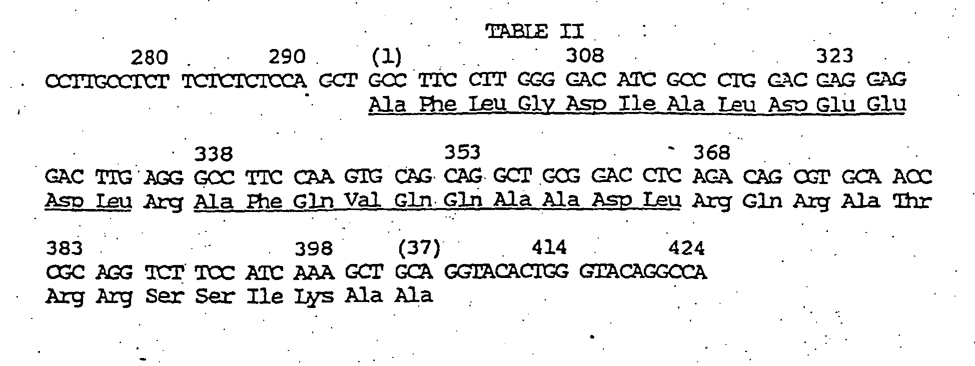

### EXAMPLE V

### Human Bone Inductive Factors

### A. hBMP-1

Because the bovine and human bone growth factor genes are presumed to be significantly homologous, the bovine bBMP-1 DNA sequence of Table II (or portions thereof) is used as a probe to screen a human genomic library. The 800bp EcoRI fragment of the bovine genomic clone is labelled with ³²P by nick-translation. A human, genomic library (Toole et al., supra) is plated on 20 plates at 40,000 recombinants per plate. Duplicate nitrocellulose filter replicas are made of each plate and hybridized to the nick-translated probe in 5 X SSC, 5 X Denhardt's, 100ug/ml denatured salmon sperm DNA, 0.1% SDS (the standard hybridization solution) at 50 degrees centigrade for approximately 14 hours. The filters are then washed in 1 X SSC, 0.1% SDS at 50 degrees centigrade and subjected to autoradiography. Five duplicate positives are isolate and plaque purified. DNA is obtained from a plate lysate of one of these recombinant bacteriophage, designated LP-H1. LP-H1 was deposited with the ATCC on March 6, 1987, under accession number 40311. This clone encodes at least a portion of the human genomic bone growth factor called hBMP-1. The hybridizing region of LP-H1 is localized to a 2.5kb XbaI/HindIII restriction fragment.

The partial DNA sequence and derived amino acid sequence of lambda LP-H1 are shown below in Table V. The peptide sequence from this clone is 37 amino acids in length and is encoded by the DNA sequence from nucleotide #3440 through nucleotide #3550. The coding sequence of Table V is flanked by approximately 28 nucleotides (a presumptive 5' noncoding sequence) as well as approximately 19 nucleotides (a presumptive 3' noncoding sequence. A comparison of the bBMP-1 sequence of Table II with the hBMP-1 genomic sequence of Table V indicates the significant homology between the two.

Because the size of coding regions and the positions of noncoding regions is generally conserved in homologous genes of different species, the locations of the coding and noncoding regions of the bone inductive factor genes may be identified. Regions of homology between the two species' genes, flanked by RNA processing signals at homologous sites, indicate a coding region.

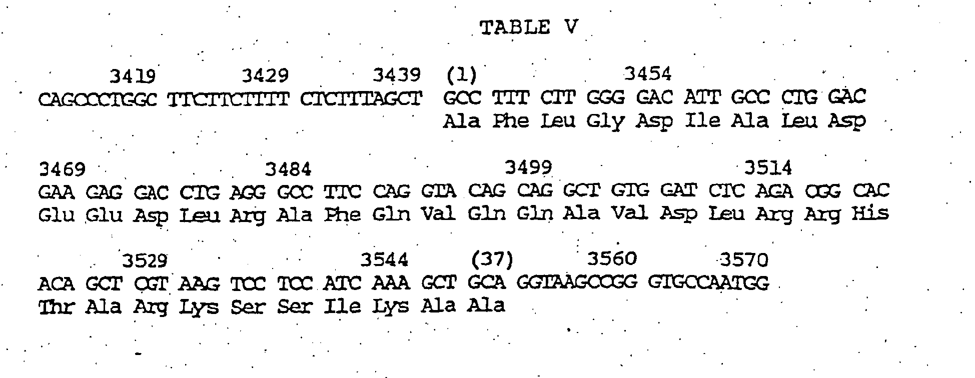

A probe specific for the human coding sequence given in Table V is used to identify a human cell line or tissue which synthesizes bone inductive factor. The probe is made according to the following method. Two oligonucleotides having the following sequences:
(a) GGGAATTCTGCCTTTCTTGGGGACATTGCCCTGGACGAAGAGGACCTGAG
(b) CGGGATCCGTCTGAGATCCACAGCCTGCTGTACCTGGAAGGCCCTCAGG
are synthesized on an automated synthesizer, annealed, extended using the Klenow fragment of E. coli DNA polymerase I, digested with the restriction enzymes Eco RI and Bam HI, and inserted into an M13 vector. A single-stranded ³²P-labeled probe is then from template preparation of this subclone by standard techniques. Polyadenylated RNAs from various cell and tissue sources are electrophoresed on formaldehyde-agarose gels and transferred to nitrocellulose by the method of Toole et al., supra. The probe is then hybridized to the nitrocellulose blot in 50% formamide, 5 X SSC, 0.1% SDS, 40 mM sodium phosphate pH 6.5, 100 ug/ml denatured salmon sperm DNA, and 5 mM vanadyl ribonucleosides at 42° C overnight and washed at 65° C in 0.2. X SSC, 0.1% SDS. Hollowing autoradiography, the lane containing RNA from the human osteosarcoma cell line U-2 OS contain hybridizing bands corresponding to RNA species of approximately 4.3 and 3.0 kb.

DNA is synthesized from U-2 OS polyadenylated RNA and cloned into lambda gt10 by established techniques (Toole et al., supra). 20,000 recombinants from this library are plated on each of 50 plates. Duplicate nitrocellulose replicas are made of the plates. The above described oligonucleotides are kinased with ³²P-gamma-ATP and hybridized to the two sets of replicas at centigrade in standard hybridization solution overnight. The filters are then washed in 1 X SSC, 0.1% SDS at 55° centigrade and subjected to autoradiography. One duplicate positive, designated lambda U2OS-1, is plaque purified. Lambda U2OS-1 was deposited with the ATCC on June 16, 1987 under accession number 40343.

The entire nucleotide sequence and derived amino acid sequence of the insert of lambda U2OS-1 is given in Table VI. This cDNA clone encodes a Met followed by a hydrophobic leader sequence characteristic of a secreted protein, and contains a stop codon at nucleotide positons 2226 - 2228. This clone contains an open reading frame of 2190bp, encoding a protein of 730 amino acids with a molecular weight og 83kd based on this amino acid sequence. The clone contains sequence identical to the coding region given in Table V. This protein is contemplated to represent a primary translation product which is cleaved upon secretion to produce the hBMP-1 protein. This clone is therefore a cDNA for hBMP-1 corresponding to human gene fragment contained in the genomic hBMP-1 sequence lambda LP-H1. It is noted that amino acids #550 to #590 of BMP-1 are homologous to epidermal growth factor and the "growth factor" domains of Protein C, Factor X and Factor IX.

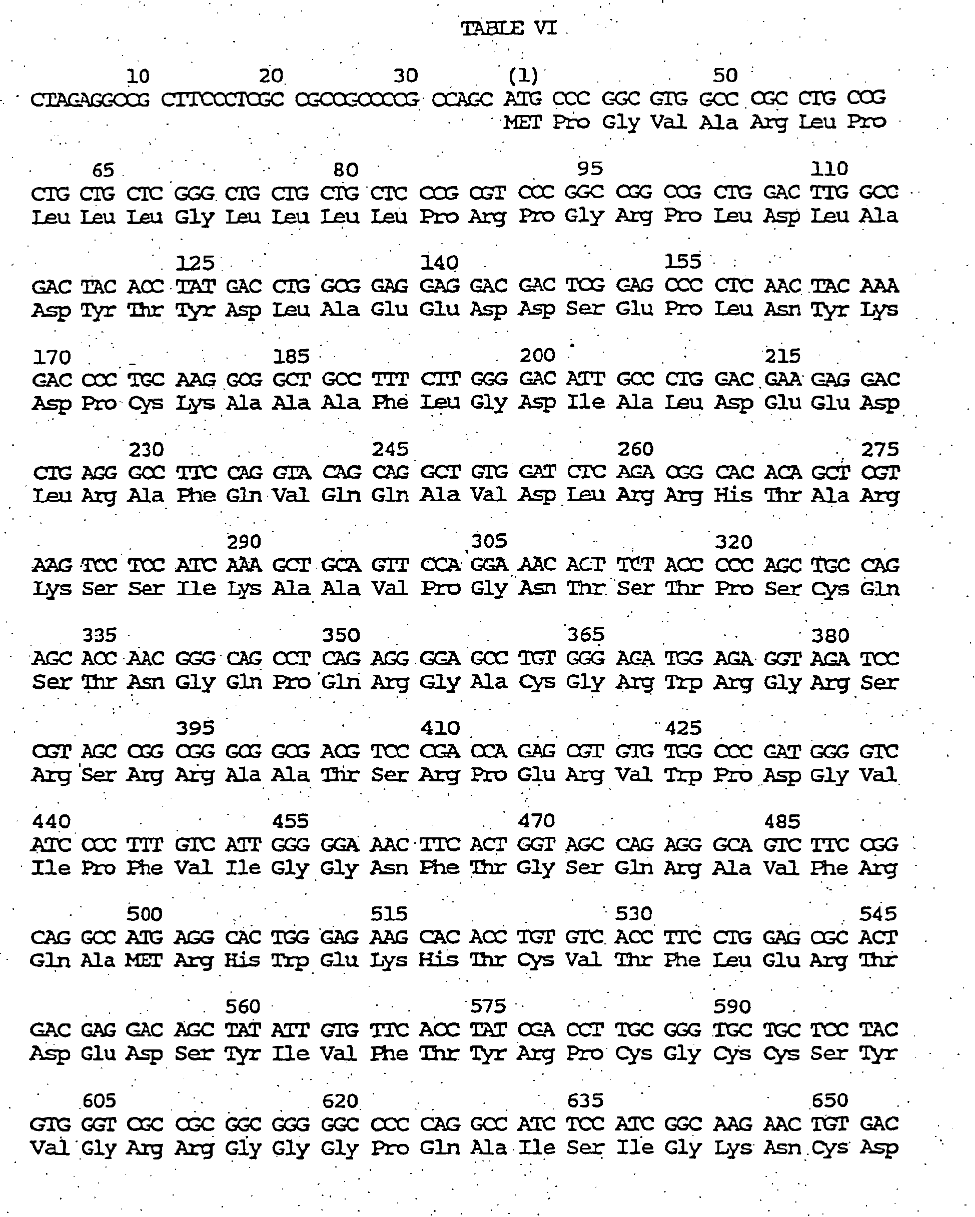

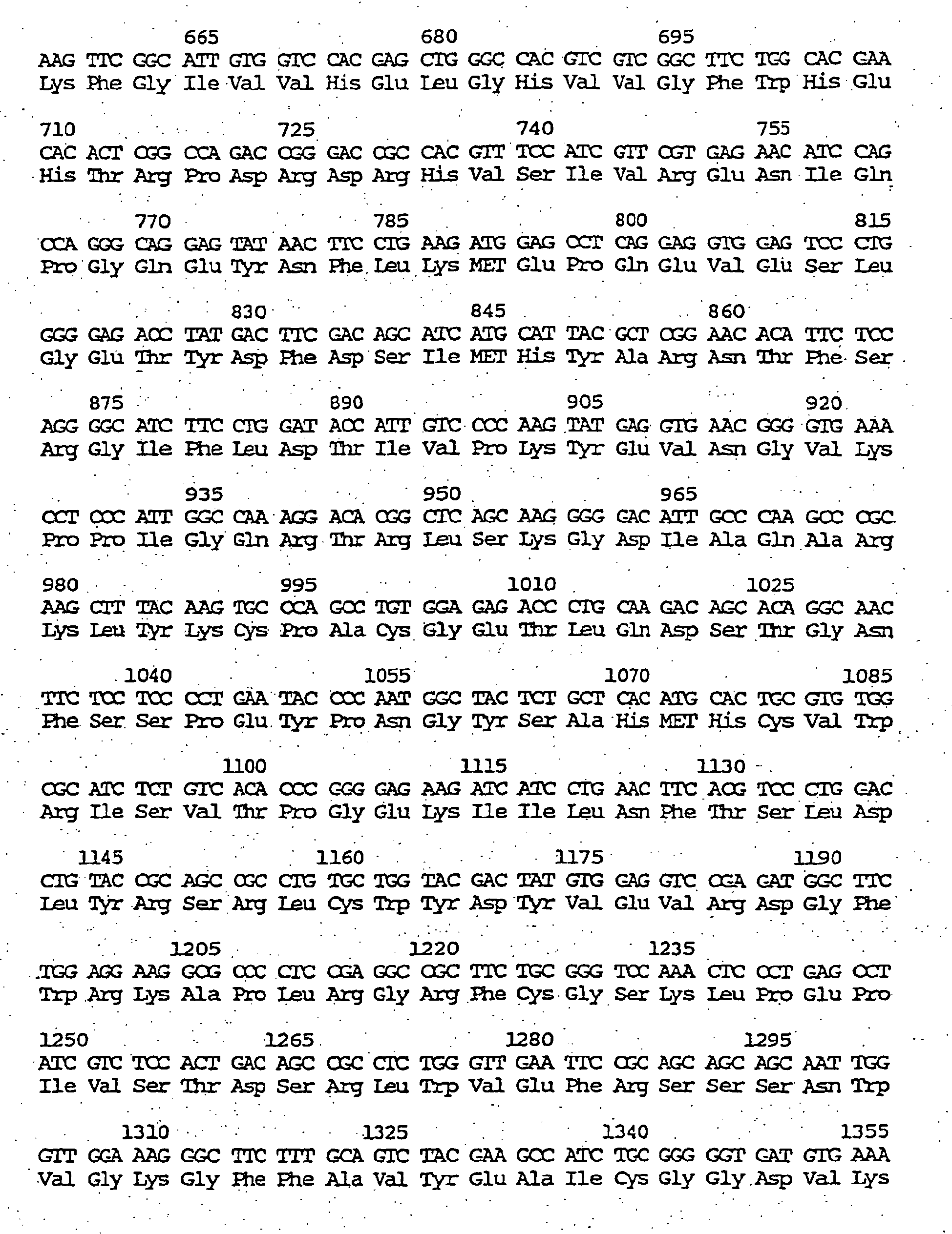

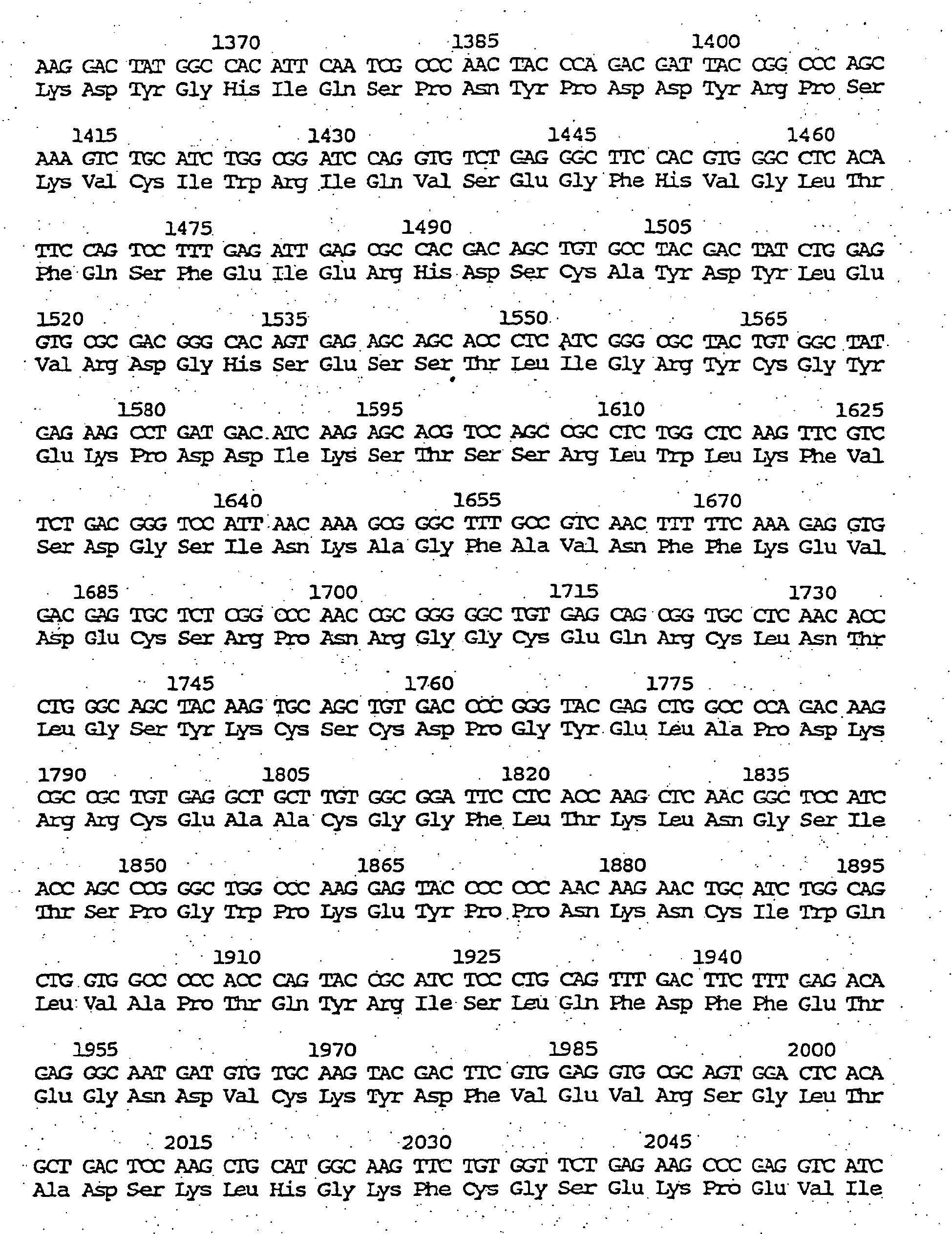

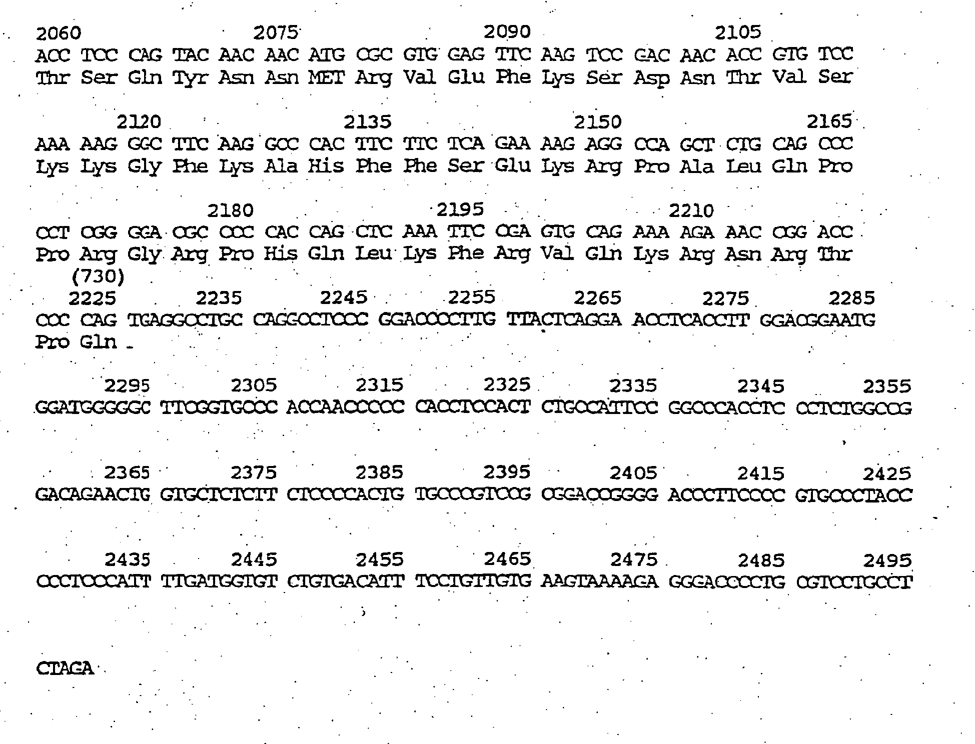

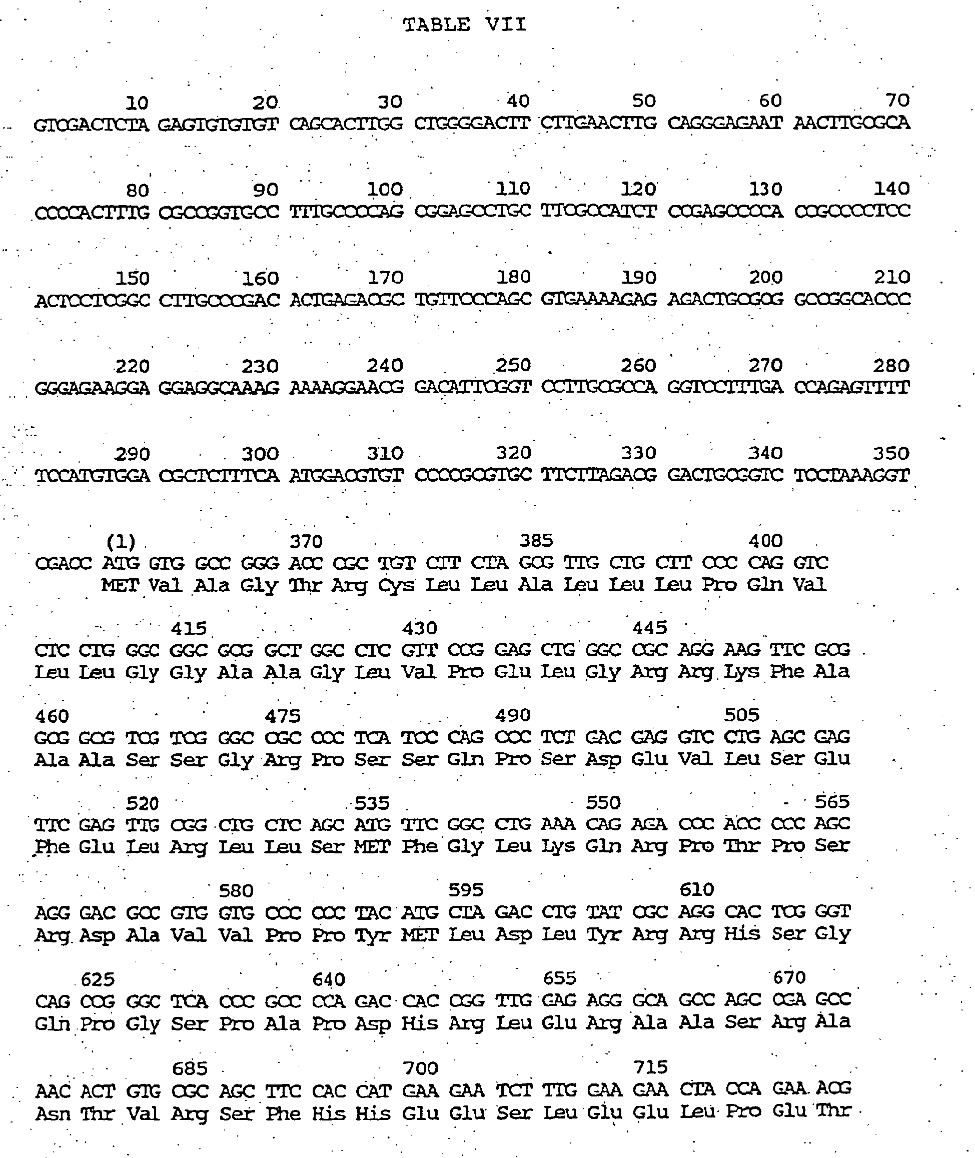

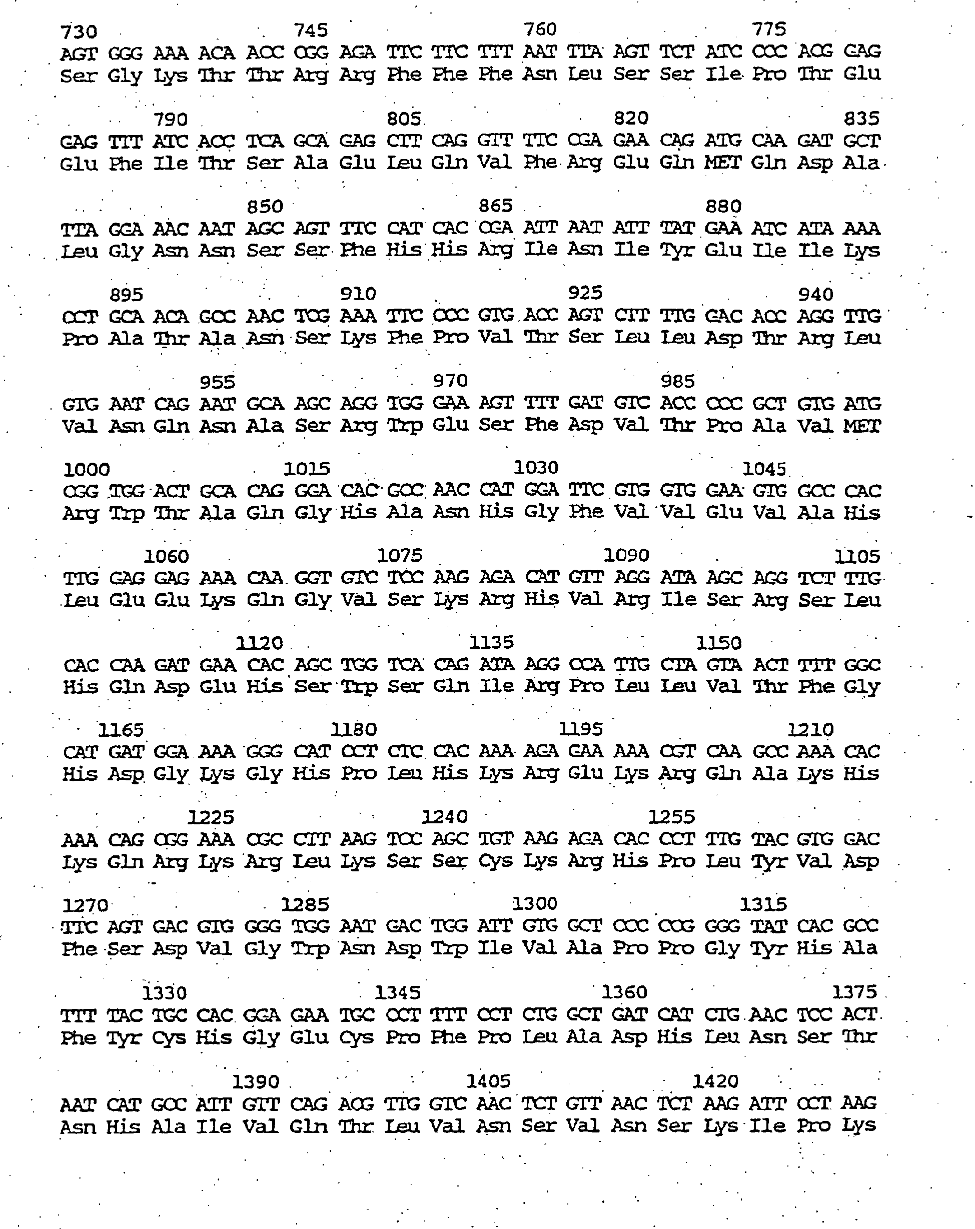

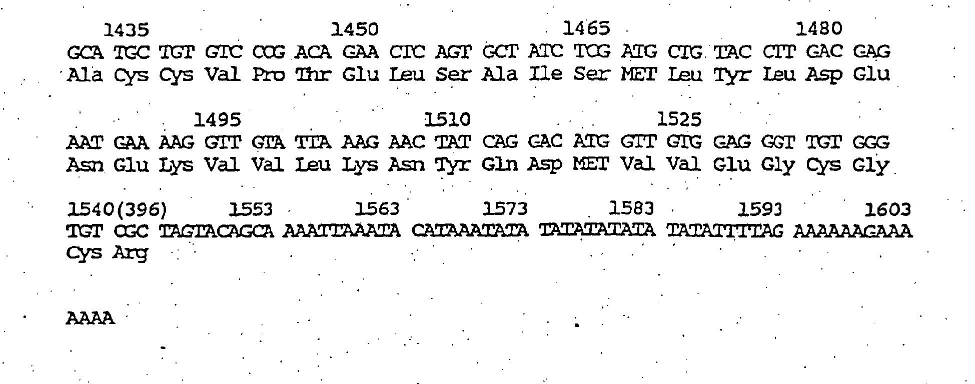

The procedures described above may similarly be employed to isolate other species' bone inductive factor of interest by utilizing the bovine bone inductive factor and/or human bone inductive factor as a probe source. Such other species' bone inductive factor may find similar utility in, inter alia, fracture repair.

### EXAMPLE VI

### Expression of Bone Inductive Factors.

In order to produce bovine, human or other mammalian bone inductive factors, the DNA encoding it is transferred into an appropriate expression vector and introduced into mammalian cells by conventional genetic engineering techniques.

One skilled in the art can constructs mammalian expression vectors by employing the sequence of Tables II-VIII or other modified sequences and known vectors, such as pCD [Okayama et al., Mol. Cell Biol., 2:161-170 (1982)] and pJL3, pJL4 [Gough et al., EMBO J., 4:645-653-(1985)]. The transformation of these vectors into appropriate host cells can result in expression of osteoinductive factors. One skilled in the art could manipulate the sequences of Tables II-VIII by eliminating or replacing the mammalian regulators sequences flanking the coding sequence with bacterial sequences to create bacterial vectors for intracellular or extracellular expression by bacterial cells. For example, the coding sequences could be further manipulated (e.g, ligated to other known linkers or modified by deleting non-coding sequences there-from or altering nucleotides therein by other know techniques). The modified bone inductive factor coding sequence could then be inserted into a known bacterial vector using procedures such as described in T. Taniguchi et al., Proc. Natl Acad. Sci. USA, 77:5230-5233 (1980). This exemplary bacterial vector could then be transformed into bacterial host cells and bone inductive factor expressed thereby. For a strategy for producing extracellular expression of bone inductive factor in bacterial cells., see, e.g. European patent application EPA 177,343.

similar manipulations can be performed for the construction of an insect vector [See, e.g. procedures described in published European patent application 155, 476] for expression in insect cells. A yeast vector could also be constructed employing yeast regulatory sequences for intracellular or extracellular expression of the factors of the present invention by yeast cells. [See, e.g., procedures described in published PCT application WO86/00639 and European patent application EPA 123,289].

A method for producing high levels of an osteoinductive factor of the invention from mammalian cells involves the construction of cells containing multiple copies of the heterologous bone inductive factor gene. The heterologous gene can be linked to an amplifiable marker, e.g. the dihydrofolate reductase (DHFR) gene for which cells containing increased gene copies can be selected for propagation in increasing concentrations of methotrexate (MTX) according to the procedures of Kaufman and Sharp, J. Mol. Biol., 159:601-629 (1982). This approach can be employed with a number of different cell types.

For example, a plasmid containing a DNA sequence for a bone inductive factor of the invention in operative association with other plasmid sequences enabling expression thereof and the DHFR expression plasmid pAdA26SV(A)3 [Kaufman and Sharp, Mol. Cell. Biol., 2:1304 (1982)] can be co-introduced into DHFR-deficient CHO cells, DUKX-BII, by calcium phosphate coprecipitation and transfection. DHFR expressing transformants are selected for growth in alpha media with dialyzed fetal calf serum, and subsequently selected for amplification by growth in increasing concentrations of MTX (sequential steps in 0.02, 0.2, 1.0 and 5uM MTX) as described in Kaufman et al., Mol Cell Biol., 5:1750 (1983). Transformants are cloned, and biologically active bone inductive factor expression is monitored by rat bone formation assay. Bone inductive factor expression should increase with increasing levels of MTX resistance. Similar procedures can be followed to produce other bone inductive factors.

Alternatively, the human gene is expressed directly, as described above. Active bone inductive factor may be produced in bacteria or yeast cells. However the presently preferred expression system for biologically active recombinant human bone inductive factor is stably transformed CHO cells.

As one specific example, to produce the human bone inductive factor (hBMP-1) of Example V, the insert of U2OS-1 is released from the vector arms by digestion with Sal I and subcloned into the mammalian expression vector pMT2CX digested with Xho I. Plasmid DNA. from this subclone is transfected into COS cells by the DEAE-dextran procedure [Sompayrac and Danna PNAS 78:7575-7578 (1981); Luthman and Magnusson, Nucl.Acids Res. 11: 1295-1308 (1963)]. Serum-free 24 hr. conditioned medium is collected from the cells starting 40-70 hr. post-transfection.

The mammalian expression vector pMT2 Cla-Xho (pMT2 CX) is a derivative of p91023 (b) (Wong et al., Science 228: 810-815, 1985) differing from the latter in that it contain the ampicillin resistance gene in place of the tetracycline resistance gene and further contains a XhoI site for insertion of cDNA clones. The functional elements of pMT2 Cla-Xho have been described (Kaufman, R.J., 1985, Proc. Natl. Acad. Sci. USA 82:689-693) and included the adenovirus VA genes, the SV40 origin of replication including the 72 bp enhancer, the adenovirus major late promoter including a 5' splice site and the majority of the adenovirus tripartite leader sequence present on adenovirus late mRNAs, a 3' splice acceptor site, a DHFR insert, the SV40 early polyadenylation site (SV40), and pBR322 sequences needed for propagation in E. coli.

Plasmid pMT2 Cla-Xho is obtained by EcoRI digestion of pMT2-VWF, which has been deposited with the American Type Culture Collection (ATCC), Rockville, MD (USA) under accession number ATCC 67122. EcoRI digestion excises the cDNA inset present in pMT2-VWF, yielding pMT2 in linear form which can be ligated and used to transform E. coli HB 101 or DH-5 to ampicillin resistance. Plasmid pMT2 DNA can be prepared by conventional methods. pMT2CX is then constructed by digesting pMT2 with Eco RV and xbaI, treating the digested DNA with Klenow fragment of DNA polymerase I, and ligating Cla linkers (NEBiolabs, CATCGATG). This removes bases 2266 to 2421 starting from the Hind III site near the SV40 origin of replication and enhancer sequences of pMT2. Plasmid DNA is then digested with EcoRI, blunted as above, and ligated to an EcoRI adapter,
5' PO₄-AATTCCTCGAGAGCT 3'
3' GGAGCTCTCGA 5'
digested with XhoI, and ligated, yielding pMT2 Cla-Xho, which may then be used to transform E. coli to ampicillin resistance. Plasmid pMT2 Cla-Xho DNA may be prepared by conventional methods.

### Example VII

### Biological Activity of Expressed Bone Inductive Factor

### A. BMP-1

To measure the biological activity of the expressed bone inductive factor. (hBMP-1) obtained in Example VI above. The factor is partially purified on a Heparin Sepharose column. 4 ml of transfection supernatant from one 100 mm dish is concentrated approximately 10 fold by ultrafiltration on a YM 10 membrane and then dialyzed against 20mM Tris, 0.15 M NaCl, pH 7.4 (starting buffer). This material is then applied to a 1.1 ml Heparin Sepharose column in starting buffer. Unbound proteins are removed by an 8 ml wash of starting buffer, and bound proteins, including BMP-1, are desorbed by a 3-4 ml wash of 20 mM Tris, 2.0 M NaCl, pH 7.4.

The proteins bound by the Heparin column are concentrated approximately 10-fold on a Centricon 10 and the salt reduced by diafiltration with 0.1% trifluoroacetic acid. The appropriate amount or this solution is mixed with 20 mg of rat matrix and then assayed for in vivo bone and cartilage formation as previously described in Example III. A mock transfection supernatant fractionation is used as a control.

The implants containing rat matrix to which specific amounts of human BMP-1 have been added are removed from rats alter seven days and processed for histological evaluation. Representative sections from each implant are stained for the presence of new bone mineral with von Kossa and acid fuschin, and for the presence of cartilage-specific matrix formation using toluidine blue. The types of cells present within the section, as well as the extend to which these cells display phenotype are evaluated.

Addition of human BMP-1 to the matrix material resulted in formation of cartilage-like nodules at 7 days post implantation. The chondroblast-type cells were recognizable by shape and expression of metachromatic matrix. The amount of activity observed for human BMP-1 was' dependent upon the amount of human BMP-1 protein added to the matrix. Table IX illustrate the dose-response relationship of human BMP-1 protein to the amount of bone induction observed.

**Table IX**

| | | |
|---|---|---|
| IMPLANT NUMBER | AMOUNT USED | HISTOLOGICAL SCORE |
| | (equivalent of ml transfection media) | |
| 876-134-1 | 10 BMP-1 | C+2 |
| | | |
| 876-134-2 | 3 BMP-1 | C+1 |
| | | |
| 87.6-13.4-3 | 1 BMP-1 | C +/- |
| | | |
| 876-134-4 | 10 MOCK | C - |
| | | |
| 876-134-5 | 3 MOCK | C - |
| | | |
| 876-134-6 | 1 MOCK | C - |

| | | |
|---|---|---|
| Cartilage (c) activity was scored on a scale from O(-) to 5. | | |

similar levels of activity are seen in the Heparin Sepharose fractionated COS cell extracts. Partial purification is accomplished in a similar manner as described above except that 6 M urea is included in all the buffers. Further, in a rat bone formation assay as described above, BMP-2 has similarly demonstrated chondrogenic activity.

The procedures described above may be employed to isolate other bone inductive factors of interest by utilizing the bovine bone inductive factors and/or human bone inductive factors as a probe source. Such other bone inductive factors may find similar utility in, inter alia, fracture repair.

The foregoing descriptions detail presently preferred embodiments of the present invention. Numerous modifications and variations in practice thereof are expected to occur to those skilled in the art upon consideration of these descriptions. Those modifications and variations are believed to be encompassed within the claims appended hereto.

## Claims

1. A gene encoding human BMP-1 comprising the following DNA sequence:

10          20          30          (1)              50
CTAGAGGCCG CTTCCCTCGC CGCCGCCCCG CCAGC ATG CCC GGC GTG GCC CGC CTG CCG
                                       MET Pro Gly Val Ala Arg Leu Pro

    65              80              95              110
CTG CTG CTC GGG CTG CTG CTG CTC CCG CGT CCC GGC CGG CCG CTG GAC TTG GCC
Leu Leu Leu Gly Leu Leu Leu Leu Pro Arg Pro Gly Arg Pro Leu Asp Leu Ala

            125             140             155
GAC TAC ACC TAT GAC CTG GCG GAG GAG GAC GAC TCG GAG CCC CTC AAC TAC AAA
Asp Tyr Thr Tyr Asp Leu Ala Glu Glu Asp Asp Ser Glu Pro Leu Asn Tyr Lys

170             185             200             215
GAC CCC TGC AAG GCG GCT GCC TTT CTT GGG GAC ATT GCC CTG GAC GAA GAG GAC
Asp Pro Cys Lys Ala Ala Ala Phe Leu Gly Asp Ile Ala Leu Asp Glu Glu Asp

        230             245             260             275
CTG AGG GCC TTC CAG GTA CAG CAG GCT GTG GAT CTC AGA CGG CAC ACA GCT CGT
Leu Arg Ala Phe Gln Val Gln Gln Ala Val Asp Leu Arg Arg His Thr Ala Arg

            290             305             320
AAG TCC TCC ATC AAA GCT GCA GTT CCA GGA AAC ACT TCT ACC CCC AGC TGC CAG
Lys Ser Ser Ile Lys Ala Ala Val Pro Gly Asn Thr Ser Thr Pro Ser Cys Gln

    335             350             365             380
AGC ACC AAC GGG CAG CCT CAG AGG GGA GCC TGT GGG AGA TGG AGA GGT AGA TCC
Ser Thr Asn Gly Gln Pro Gln Arg Gly Ala Cys Gly Arg Trp Arg Gly Arg Ser

            395             410             425
CGT AGC CGG CGG GCG GCG ACG TCC CGA CCA GAG CGT GTG TGG CCC GAT GGG GTC
Arg Ser Arg Arg Ala Ala Thr Ser Arg Pro Glu Arg Val Trp Pro Asp Gly Val

440             455             470             485
ATC CCC TTT GTC ATT GGG GGA AAC TTC ACT GGT AGC CAG AGG GCA GTC TTC CGG
Ile Pro Phe Val Ile Gly Gly Asn Phe Thr Gly Ser Gln Arg Ala Val Phe Arg

        500             515             530             545
CAG GCC ATG AGG CAC TGG GAG AAG CAC ACC TGT GTC ACC TTC CTG GAG CGC ACT
Gln Ala MET Arg His Trp Glu Lys His Thr Cys Val Thr Phe Leu Glu Arg Thr

            560             575             590
GAC GAG GAC AGC TAT ATT GTG TTC ACC TAT CGA CCT TGC GGG TGC TGC TCC TAC
Asp Glu Asp Ser Tyr Ile Val Phe Thr Tyr Arg Pro Cys Gly Cys Cys Ser Tyr

    605             620             635             650
CTG GGT CGC CGC GGC GGG GGC CCC CAG GCC ATC TCC ATC GGC AAG AAC TGT GAC
Val Gly Arg Arg Gly Gly Gly Pro Gln Ala Ile Ser Ile Gly Lys Asn Cys Asp

665 680 695
AAG TTC GGC ATT GTG GTC CAC GAG CTG GGC CAC GTC GTC GGC TTC TGG CAC GAA
Lys Phe Gly Ile Val Val His Glu Leu Gly His Val Val Gly Phe Trp His Glu

710 725 740 755
CAC ACT CGG CCA GAC CGG GAC CGC CAC GTT TCC ATC GTT CGT GAG AAC ATC CAG
His Thr Arg Pro Asp Arg Asp Arg His Val Ser Ile Val Arg Glu Asn Ile Gln

770 785 800 815
CCA GGG CAG GAG TAT AAC TTC CTG AAG ATG GAG CCT CAG GAG GTG GAG TCC CTG
Pro Gly Gln Glu Tyr Asn Phe Leu Lys MET Glu Pro Gln Glu Val Glu Ser Leu

830 845 860
GGG GAG ACC TAT GAC TTC GAC AGC ATC ATG CAT TAC GCT CGG AAC ACA TTC TCC
Gly Glu Thr Tyr Asp Phe Asp Ser Ile MET His Tyr Ala Arg Asn Thr Phe Ser

875 890 905 920
AGG GGC ATC TTC CTG GAT ACC ATT GTC CCC AAG TAT GAG GTG AAC GGG GTG AAA
Arg Gly Ile Phe Leu Asp Thr Ile Val Pro Lys Tyr Glu Val Asn Gly Val Lys

935 950 965
CCT CCC ATT GGC CAA AGG ACA CGG CTC AGC AAG GGG GAC ATT GCC CAA GCC CGC
Pro Pro Ile Gly Gln Arg Thr Arg Leu Ser Lys Gly Asp Ile Ala Gln Ala Arg

980 995 1010 1025
AAG CTT TAC AAG TGC CCA GCC TGT GGA GAG ACC CTG CAA GAC AGC ACA GGC AAC
Lys Leu Tyr Lys Cys Pro Ala Cys Gly Glu Thr Leu Gln Asp Ser Thr Gly Asn

1040 1055 1070 1085
TTC TCC TCC CCT GAA TAC CCC AAT GGC TAC TCT GCT CAC ATG CAC TGC GTG TGG
Phe Ser Ser Pro Glu Tyr Pro Asn Gly Tyr Ser Ala His MET His Cys Val Trp

1100 1115 1130
CGC ATC TCT GTC ACA CCC GGG GAG AAG ATC ATC CTG AAC TTC ACG TCC CTG GAC
Arg Ile Ser Val Thr Pro Gly Glu Lys Ile Ile Leu Asn Phe Thr Ser Leu Asp

1145 1160 1175 1190
CTG TAC CGC AGC CGC CTG TGC TGG TAC GAC TAT GTG GAG GTC CGA GAT GGC TTC
Leu Tyr Arg Ser Arg Leu Cys Trp Tyr Asp Tyr Val Glu Val Arg Asp Gly Phe

1205 1220 1235
TGG AGG AAG GCG CCC CTC CGA GGC CGC TTC TGC GGG TCC AAA CTC CCT GAG CCT
Trp Arg Lys Ala Pro Leu Arg Gly Arg Phe Cys Gly Ser Lys Leu Pro Glu Pro

1250 1265 1280 1295
ATC GTC TCC ACT GAC AGC CGC CTC TGG GTT GAA TTC CGC AGC AGC AGC AAT TGG
Ile Val Ser Thr Asp Ser Arg Leu Trp Val Glu Phe Arg Ser Ser Ser Asn Trp

1310 1325 1340 1355
GTT GGA AAG GGC TTC TTT GCA GTC TAC GAA GCC ATC TGC GGG GGT GAT GTG AAA
Val Gly Lys Gly Phe Phe Ala Val Tyr Glu Ala Ile Cys Gly Gly Asp Val Lys

1370                    1385                    1400
AAG GAC TAT GGC CAC ATT CAA TCG CCC AAC TAC CCA GAC GAT TAC CGG CCC AGC
Lys Asp Tyr Gly His Ile Gln Ser Pro Asn Tyr Pro Asp Asp Tyr Arg Pro Ser

1415                    1430                    1445                    1460
AAA GTC TGC ATC TGG CGG ATC CAG GTG TCT GAG GGC TTC CAC GTG GGC CTC ACA
Lys Val Cys Ile Trp Arg Ile Gln Val Ser Glu Gly Phe His Val Gly Leu Thr

1475                    1490                    1505
TTC CAG TCC TTT GAG ATT GAG CGC CAC GAC AGC TGT GCC TAC GAC TAT CTG GAG
Phe Gln Ser Phe Glu Ile Glu Arg His Asp Ser Cys Ala Tyr Asp Tyr Leu Glu

1520                    1535                    1550                    1565
GTG CGC GAC GGG CAC AGT GAG AGC AGC ACC CTC ATC GGC CGC TAC TGT GGC TAT
Val Arg Asp Gly His Ser Glu Ser Ser Thr Leu Ile Gly Arg Tyr Cys Gly Tyr

1580                    1595                    1610                    1625
GAG AAG CCT GAT GAC ATC AAG AGC ACG TCC AGC CGC CTC TGG CTC AAG TTC GTC
Glu Lys Pro Asp Asp Ile Lys Ser Thr Ser Ser Arg Leu Trp Leu Lys Phe Val

1640                    1655                    1670
TCT GAC GGG TCC ATT AAC AAA GCG GGC TTT GCC GTC AAC TTT TTC AAA GAG GTG
Ser Asp Gly Ser Ile Asn Lys Ala Gly Phe Ala Val Asn Phe Phe Lys Glu Val

1685                    1700                    1715                    1730
GAC GAG TGC TCT CGG CCC AAC CGC GGG GGC TGT GAG CAG CGG TGC CTC AAC ACC
Asp Glu Cys Ser Arg Pro Asn Arg Gly Gly Cys Glu Gln Arg Cys Leu Asn Thr

1745                    1760                    1775
CTG GGC AGC TAC AAG TGC AGC TGT GAC CCC GGG TAC GAG CTG GCC CCA GAC AAG
Leu Gly Ser Tyr Lys Cys Ser Cys Asp Pro Gly Tyr Glu Leu Ala Pro Asp Lys

1790                    1805                    1820                    1835
CGC CGC TGT GAG GCT GCT TGT GGC GGA TTC CTC ACC AAG CTC AAC GGC TCC ATC
Arg Arg Cys Glu Ala Ala Cys Gly Gly Phe Leu Thr Lys Leu Asn Gly Ser Ile

1850                    1865                    1880                    1895
ACC AGC CCG GGC TGG CCC AAG GAG TAC CCC CCC AAC AAG AAC TGC ATC TGG CAG
Thr Ser Pro Gly Trp Pro Lys Glu Tyr Pro Pro Asn Lys Asn Cys Ile Trp Gln

1910                    1925                    1940
CTG GTG GCC CCC ACC CAG TAC CGC ATC TCC CTG CAG TTT GAC TTC TTT GAG ACA
Leu Val Ala Pro Thr Gln Tyr Arg Ile Ser Leu Gln Phe Asp Phe Phe Glu Thr

1955                    1970                    1985                    2000
GAG GGC AAT GAT GTG TGC AAG TAC GAC TTC GTG GAG GTG CGC AGT GGA CTC ACA
Glu Gly Asn Asp Val Cys Lys Tyr Asp Phe Val Glu Val Arg Ser Gly Leu Thr

2015                    2030                    2045
GCT GAC TCC AAG CTG CAT GGC AAG TTC TGT GGT TCT GAG AAG CCC GAG GTC ATC
Ala Asp Ser Lys Leu His Gly Lys Phe Cys Gly Ser Glu Lys Pro Glu Val Ile

2060 2075 2090 2105
ACC TCC CAG TAC AAC AAC ATG CGC GTG GAG TTC AAG TCC GAC AAC ACC GTG TCC
Thr Ser Gln Tyr Asn Asn MET Arg Val Glu Phe Lys Ser Asp Asn Thr Val Ser

2120 2135 2150 2165
AAA AAG GGC TTC AAG GCC CAC TTC TTC TCA GAA AAG AGG CCA GCT CTG CAG CCC
Lys Lys Gly Phe Lys Ala His Phe Phe Ser Glu Lys Arg Pro Ala Leu Gln Pro

2180 2195 2210
CCT CGG GGA CGC CCC CAC CAG CTC AAA TTC CGA GTG CAG AAA AGA AAC CGG ACC
Pro Arg Gly Arg Pro His Gln Leu Lys Phe Arg Val Gln Lys Arg Asn Arg Thr
(730)
2225 2235 2245 2255 2265 2275 2285
CCC CAG TGAGCCCTGC CAGGCCTCCC GGACCCCCTG TTACTCAGGA ACCTCACCTT GGACGGAATG
Pro Gln

2295 2305 2315 2325 2335 2345 2355
GGATGGGGGC TTCGGTGCCC ACCAACCCCC CACCTCCACT CTGCCATTCC GGCCCACCTC CCTCTGGC[?]G

2365 2375 2385 2395 2405 2415 2425
GACAGAACTG GTGCTCTCTT CTCCCCACTG TGCCCTGTCC GCGACCCGGG ACCCTTCCCC GTGCCCTACC

2435 2445 2455 2465 2475 2485 2495
CCCTCCCATT TTGATGGTGT CTGTGACATT TCCTGTTGTG AAGTAAAAGA GGGACCCCTG CGTCCTGCCT

CCAGA

2. A gene encoding human BMP-1 having the amino acid sequence given in claim 1.

3. A gene encoding the BMP-1 amino acid sequence of claim 1 and comprising DNA sequences:
(a) which differ from a DNA sequence of claim 1 in codon sequence due to the degeneracy of the genetic code; or
(b) which hybridise with a DNA sequence of claim 1 or section (a), above under stringent hybridisation conditions.

4. The DNA sequence of claim 3, which is a genomic DNA sequence.

5. The DNA sequence of claim 3, which is a cDNA sequence.

6. A gene encoding bovine BMP-1 comprising the following DNA sequence:

280 . 290 (1) 308 323
CCTTGCCTCT TCTCTCTCCA GCT GCC TTC CTT GGG GAC ATC GCC CTG GAC GAG GGG
Ala Phe Leu Gly Asp Ile Ala Leu Asp Glu Gly

338 353 . 368
GAC TTG AGG GCC TTC CAA GTG CAG CAG GCT GCG GAC CTC AGA CAG CGT GCA ACC
Asp Leu Arg Ala Phe Gln Val Gln Gln Ala Ala Asp Leu Arg Gln Arg Ala Thr

383 . 398 (37) 414 424
CGC AGG TCT TCC ATC AAA GCT GCA GGTACACTGG GTACAGGCCA
Arg Arg Ser Ser Ile Lys Ala Ala

7. A gene encoding bovine BMP- 1 containing the amino acid sequence of claim 6.

8. A gene encoding a the BMP-1 amino acid sequence of claim 6 and comprising DNA sequences:
(a) which differ from a DNA sequence of claim 6 in codon sequence due to the degeneracy of the genetic code; or
(b) which hybridise with a DNA sequence of claim 6 or section (a), above under stringent hybridisation conditions.

9. The DNA sequence of claim 8, Which is a genomic DNA sequence.

10. The DNA sequence of claim 8, which is a cDNA sequence.

11. A vector containing the gene or DNA sequence of any one of claims 1 to 10 in operative association with an expression control sequence.

12. A cell transformed with a vector of claim 11.

13. The cell of claim 12 which is a mammalian cell, a bacterial cell, an insect cell or a yeast cell,

14. A protein exhibiting properties of BMP-1 which is encoded by the gene or DNA sequence of any one of claims 1 to 10.

15. A process for producing the protein of claim 14, comprising the steps of culturing in a suitable culture medium the cell of claim 13 and isolating said protein from said culture medium.

16. A pharmaceutical composition comprising the protein of claim 14 and a pharmaceutically acceptable vehicle.

17. The pharmaceutical composition of Claim 16, further comprising a matrix capable of delivering the composition to the site of the bone or cartilage defect and providing a structure for inducing bone or cartilage formation.

18. The pharmaceutical composition of claim 17, wherein said matrix comprises hydroxyapatite, collagen, polylactic acid or tricalcium phosphate.

19. Use of a protein of claim 14 for the preparation of a pharmaceutical composition for inducing bone or cartilage formation.

## Patentansprüche

1. Gen, das humanes BMP-1 kodiert, das die folgende DNA-Sequenz umfasst:

10 20 30 (1) 50
CTAGAGGCCG GTTCCCTCGC CGCCGCCCCG CCAGC ATG CCC GGC GTG GCC CGC CTG CCG
MET Pro Gly Val Ala Arg Leu Pro

65 80 95 110
CTG CTG CTC GGG CTG CTG CTG CTC CCG CGT CCC GGC CGG CCG CTG GAC TTG GCC
Leu Leu Leu Gly Leu Leu Leu Leu Pro Arg Pro Gly Arg Pro Leu Asp Leu Ala

125 140 155
GAC TAC ACC TAT GAC CTG GCG GAG GAG GAC GAC TCG GAG CCC CTC AAC TAC AAA
Asp Tyr Thr Tyr Asp Leu Ala Glu Glu Asp Asp Ser Glu Pro Leu Asn Tyr Lys

170 185 200 215
GAC CCC TGC AAG GCG GCT GCC TTT CTT GGG GAC ATT GCC CTG GAC GAA GAG GAC
Asp Pro Cys Lys Ala Ala Ala Phe Leu Gly Asp Ile Ala Leu Asp Glu Glu Asp

230 245 260 275
CTG AGG GCC TTC CAG GTA CAG CAG GCT GTG GAT CTC AGA CGG CAC ACA GCT CGT
Leu Arg Ala Phe Gln Val Gln Gln Ala Val Asp Leu Arg Arg His Thr Ala Arg

290 305 320
AAG TCC TCC ATC AAA GCT GCA GTT CCA GGA AAC ACT TCT ACC CCC AGC TGC CAG
Lys Ser Ser Ile Lys Ala Ala Val Pro Gly Asn Thr Ser Thr Pro Ser Cys Gln

335 350 365 380
AGC ACC AAC GGG CAG CCT CAG AGG GGA GCC TGT GGG AGA TGG AGA GGT AGA TCC
Ser Thr Asn Gly Gln Pro Gln Arg Gly Ala Cys Gly Arg Trp Arg Gly Arg Ser

395 410 425
CGT AGC CGG CGG GCG GCG ACG TCC CGA CCA GAG CGT GTG TGG CCC GAT GGG GTC
Arg Ser Arg Arg Ala Ala Thr Ser Arg Pro Glu Arg Val Trp Pro Asp Gly Val

440 455 470 485
ATC CCC TTT GTC ATT GGG GGA AAC TTC ACT GGT AGC CAG AGG GCA GTC TTC CGG
Ile Pro Phe Val Ile Gly Gly Asn Phe Thr Gly Ser Gln Arg Ala Val Phe Arg

500 515 530 545
CAG GCC ATG AGG CAC TGG GAG AAG CAC ACC TGT GTC ACC TTC CTG GAG CGC ACT
Gln Ala MET Arg His Trp Glu Lys His Thr Cys Val Thr Phe Leu Glu Arg Thr

560 575 590
GAC GAG GAC AGC TAT ATT GTG TTC ACC TAT CGA CCT TGC GGG TGC TGC TCC TAC
Asp Glu Asp Ser Tyr Ile Val Phe Thr Tyr Arg Pro Cys Gly Cys Cys Ser Tyr

605 620 635 650
GTG GGT CGC CGC GGC GGG GGC CCC CAG GCC ATC TCC ATC GGC AAG AAC TGT GAC
Val Gly Arg Arg Gly Gly Gly Pro Gln Ala Ile Ser Ile Gly Lys Asn Cys Asp

665 680 695
AAG TTC GGC ATT GTG GTC CAC GAG CTG GGC CAC GTC GTC GGC TTC TGG CAC GAA
Lys Phe Gly Ile Val Val His Glu Leu Gly His Val Val Gly Phe Trp His Glu

710 725 740 755
CAC ACT CGG CCA GAC CGG GAC CGC CAC GTT TCC ATC GTT CGT GAG AAC ATC CAG
His Thr Arg Pro Asp Arg Asp Arg His Val Ser Ile Val Arg Glu Asn Ile Gln

770 785 800 815
CCA GGG CAG GAG TAT AAC TTC CTG AAG ATG GAG CCT CAG GAG GTG GAG TCC CTG
Pro Gly Gln Glu Tyr Asn Phe Leu Lys MET Glu Pro Gln Glu Val Glu Ser Leu

830 845 860
GGG GAG ACC TAT GAC TTC GAC AGC ATC ATG CAT TAC GCT CGG AAC ACA TTC TCC
Gly Glu Thr Tyr Asp Phe Asp Ser Ile MET His Tyr Ala Arg Asn Thr Phe Ser

875 890 905 920
AGG GGC ATC TTC CTG GAT ACC ATT GTC CCC AAG TAT GAG GTG AAC GGG GTG AAA
Arg Gly Ile Phe Leu Asp Thr Ile Val Pro Lys Tyr Glu Val Asn Gly Val Lys

935 950 965
CCT CCC ATT GGC CAA AGG ACA CGG CTC AGC AAG GGG GAC ATT GCC CAA GCC CGC
Pro Pro Ile Gly Gln Arg Thr Arg Leu Ser Lys Gly Asp Ile Ala Gln Ala Arg

980 995 1010 1025
AAG CTT TAC AAG TGC CCA GCC TGT GGA GAG ACC CTG CAA GAC AGC ACA GGC AAC
Lys Leu Tyr Lys Cys Pro Ala Cys Gly Glu Thr Leu Gln Asp Ser Thr Gly Asn

1040 1055 1070 1085
TTC TCC TCC CCT GAA TAC CCC AAT GGC TAC TCT GCT CAC ATG CAC TGC GTG TGG
Phe Ser Ser Pro Glu Tyr Pro Asn Gly Tyr Ser Ala His MET His Cys Val Trp

1100 1115 1130
CGC ATC TCT GTC ACA CCC GGG GAG AAG ATC ATC CTG AAC TTC ACG TCC CTG GAC
Arg Ile Ser Val Thr Pro Gly Glu Lys Ile Ile Leu Asn Phe Thr Ser Leu Asp

1145 1160 1175 1190
CTG TAC CGC AGC CGC CTG TGC TGG TAC GAC TAT GTG GAG GTC CGA GAT GGC TTC
Leu Tyr Arg Ser Arg Leu Cys Trp Tyr Asp Tyr Val Glu Val Arg Asp Gly Phe

1205 1220 1235
TGG AGG AAG GCG CCC CTC CGA GGC CGC TTC TGC GGG TCC AAA CTC CCT GAG CCT
Trp Arg Lys Ala Pro Leu Arg Gly Arg Phe Cys Gly Ser Lys Leu Pro Glu Pro

1250 1265 1280 1295
ATC GTC TCC ACT GAC AGC CGC CTC TGG GTT GAA TTC CGC AGC AGC AGC AAT TGG
Ile Val Ser Thr Asp Ser Arg Leu Trp Val Glu Phe Arg Ser Ser Ser Asn Trp

1310 1325 1340 1355
GTT GGA AAG GGC TTC TTT GCA GTC TAC GAA GCC ATC TGC GGG GGT GAT GTG AAA
Val Gly Lys Gly Phe Phe Ala Val Tyr Glu Ala Ile Cys Gly Gly Asp Val Lys

1370 1385 1400
AAG GAC TAT GGC CAC ATT CAA TCG CCC AAC TAC CCA GAC GAT TAC CGG CCC AGC
Lys Asp Tyr Gly His Ile Gln Ser Pro Asn Tyr Pro Asp Asp Tyr Arg Pro Ser

1415 1430 1445 1460
AAA GTC TGC ATC TGG CGG ATC CAG GTG TCT GAG GGC TTC CAC GTG GGC CTC ACA
Lys Val Cys Ile Trp Arg Ile Gln Val Ser Glu Gly Phe His Val Gly Leu Thr

1475 1490 1505
TTC CAG TCC TTT GAG ATT GAG CGC CAC GAC AGC TGT GCC TAC GAC TAT CTG GAG
Phe Gln Ser Phe Glu Ile Glu Arg His Asp Ser Cys Ala Tyr Asp Tyr Leu Glu

1520 1535 1550 1565
GTG CGC GAC GGG CAC AGT GAG AGC AGC ACC CTC ATC GGG CGC TAC TGT GGC TAT
Val Arg Asp Gly His Ser Glu Ser Ser Thr Leu Ile Gly Arg Tyr Cys Gly Tyr

1580 1595 1610 1625
GAG AAG CCT GAT GAC ATC AAG AGC ACG TCC AGC CGC CTC TGG CTC AAG TTC GTC
Glu Lys Pro Asp Asp Ile Lys Ser Thr Ser Ser Arg Leu Trp Leu Lys Phe Val

1640 1655 1670
TCT GAC GGG TCC ATT AAC AAA GCG GGC TTT GCC GTC AAC TTT TTC AAA GAG GTG
Ser Asp Gly Ser Ile Asn Lys Ala Gly Phe Ala Val Asn Phe Phe Lys Glu Val

1685 1700 1715 1730
GAC GAG TGC TCT CGG CCC AAC CGC GGG GGC TGT GAG CAG CGG TGC CTC AAC ACC
Asp Glu Cys Ser Arg Pro Asn Arg Gly Gly Cys Glu Gln Arg Cys Leu Asn Thr

1745 1760 1775
CTG GGC AGC TAC AAG TGC AGC TGT GAC CCC GGG TAC GAG CTG GCC CCA GAC AAG
Leu Gly Ser Tyr Lys Cys Ser Cys Asp Pro Gly Tyr Glu Leu Ala Pro Asp Lys

1790 1805 1820 1835
CGC CGC TGT GAG GCT GCT TGT GGC GGA TTC CTC ACC AAG CTC AAC GGC TCC ATC
Arg Arg Cys Glu Ala Ala Cys Gly Gly Phe Leu Thr Lys Leu Asn Gly Ser Ile

1850 1865 1880 1895
ACC AGC CCG GGC TGG CCC AAG GAG TAC CCC CCC AAC AAG AAC TGC ATC TGG CAG
Thr Ser Pro Gly Trp Pro Lys Glu Tyr Pro Pro Asn Lys Asn Cys Ile Trp Gln

1910 1925 1940
CTG GTG GCC CCC ACC CAG TAC CGC ATC TCC CTG CAG TTT GAC TTC TTT GAG ACA
Leu Val Ala Pro Thr Gln Tyr Arg Ile Ser Leu Gln Phe Asp Phe Phe Glu Thr

1955 1970 1985 2000
GAG GGC AAT GAT GTG TGC AAG TAC GAC TTC GTG GAG GTG CGC AGT GGA CTC ACA
Glu Gly Asn Asp Val Cys Lys Tyr Asp Phe Val Glu Val Arg Ser Gly Leu Thr

2015 2030 2045
GCT GAC TCC AAG CTG CAT GGC AAG TTC TGT GGT TCT GAG AAG CCC GAG GTC ATC
Ala Asp Ser Lys Leu His Gly Lys Phe Cys Gly Ser Glu Lys Pro Glu Val Ile

2060 2075 2090 2105
ACC TCC CAG TAC AAC AAC ATG CGC GTG GAG TTC AAG TCC GAC AAC ACC GTG TCC
Thr Ser Gln Tyr Asn Asn MET Arg Val Glu Phe Lys Ser Asp Asn Thr Val Ser

2120 2135 2150 2165
AAA AAG GGC TTC AAG GCC CAC TTC TTC TCA GAA AAG AGG CCA GCT CTG CAG CCC
Lys Lys Gly Phe Lys Ala His Phe Phe Ser Glu Lys Arg Pro Ala Leu Gln Pro

2180 2195 2210
CCT CGG GGA CGC CCC CAC CAG CTC AAA TTC CGA GTG CAG AAA AGA AAC CGG ACC
Pro Arg Gly Arg Pro His Gln Leu Lys Phe Arg Val Gln Lys Arg Asn Arg Thr
(730)
2225 2235 2245 2255 2265 2275 2285
CCC CAG TGAGGCCTGC CAGGCCTCCC GGACCCCTTG TTACTCAGGA ACCTCACCTT GGACGGAATG
Pro Gln

2295 2305 2315 2325 2335 2345 2355
GGATGGGGGC TTCGGTGCCC ACCAACCCCC CACCTCCACT CTGCCATTCC GGCCCACCTC CCTCTGGCCG

2365 2375 2385 2395 2405 2415 2425
GACAGAACTG GTGCTCTCTT CTCCCCACTG TGCCCGTCCG CGGACCGGGG ACCCTTCCCC GTGCCCTACC

2435 2445 2455 2465 2475 2485 2495
CCCTCCCATT TTGATGGTGT CTGTGACATT TCCTGTTGTG AAGTAAAAGA GGGACCCCTG CGTCCTGCCT

CTAGA

2. Gen, das humanes BMP-1 kodiert, das die in Anspruch 1 angegebene Aminosäuresequenz aufweist.

3. Gen, das die BMP-1-Aminosäuresequenz nach Anspruch 1 kodiert und DNA-Sequenzen umfasst:
(a) die sich aufgrund der Degeneration des genetischen Codes von einer DNA-Sequenz nach Anspruch 1 im Hinblick auf die Codonsequenz unterscheiden oder
(b) die mit einer DNA-Sequenz nach Anspruch 1 oder nach dem oben stehenden Abschnitt (a) unter stringenten Hybridisierungsbedingungen hybridisieren.

4. DNA-Sequenz nach Anspruch 3, bei der es sich um eine genomische DNA-Sequenz handelt.

5. DNA-Sequenz nach Anspruch 3, bei der es sich um eine cDNA-Sequenz handelt.

6. Gen, das bovines BMP-1 kodiert, das die folgende DNA-Sequenz umfasst:

280 290 (1) 308 323
CCTTGCCTCT TCTCTCTCCA GCT GCC TTC CTT GGG GAC ATC GCC CTG GAC GAG GAG
Ala Phe Leu Gly Asp Ile Ala Leu Asp Glu Glu

338 353 368
GAC TTG AGG GCC TTC CAA GTG CAG CAG GCT GCG GAC CTC AGA CAG CGT GCA ACC
Asp Leu Arg Ala Phe Gln Val Gln Gln Ala Ala Asp Leu Arg Gln Arg Ala Thr

383 398 (37) 414 424
CGC AGG TCT TCC ATC AAA GCT GCA GGTACACTGG GTACAGGCCA
Arg Arg Ser Ser Ile Lys Ala Ala

7. Gen, das bovines BMP-1 kodiert, das die Aminosäuresequenz nach Anspruch 6 enthält.

8. Gen, das die BMP-1-Aminosäuresequenz nach Anspruch 6 kodiert und DNA-Sequenzen umfasst:
(a) die sich aufgrund der Degeneration des genetischen Codes von einer DNA-Sequenz nach Anspruch 6 im Hinblick auf die Codonsequenz unterscheiden oder
(b) die mit einer DNA-Sequenz nach Anspruch 6 oder nach dem oben stehenden Abschnitt (a) unter stringenten Hybridisierungsbedingungen hybridisieren.

9. DNA-Sequenz nach Anspruch 8, bei der es sich um eine genomische DNA-Sequenz handelt.

10. DNA-Sequenz nach Anspruch 8, bei der es sich um eine cDNA-Sequenz handelt.

11. Vektor, der das Gen oder die DNA-Sequenz nach einem der Ansprüche 1 bis 10 in operativer Assoziation mit einer Expressionskontrollsequenz enthält.

12. Zelle, die mit einem Vektor nach Anspruch 11 transformiert wurde.

13. Zelle nach Anspruch 12, bei der es sich um eine Säugerzelle, eine Bakterienzelle, eine Insektenzelle oder eine Hefezelle handelt.

14. Protein, das Eigenschaften von BMP-1 zeigt, das von dem Gen oder der DNA-Sequenz nach einem der Ansprüche 1 bis 10 kodiert wird.

15. Verfahren zum Produzieren des Proteins nach Anspruch 14, das die Schritte des Kultivierens der Zelle nach Anspruch 13 in einem geeigneten Nährmedium und des Isolierens des Proteins aus dem Nährmedium umfasst.

16. Pharmazeutische Zusammensetzung, die das Protein nach Anspruch 14 und einen pharmazeutisch unbedenklichen Trägerstoff umfasst.

17. Pharmazeutische Zusammensetzung nach Anspruch 16, die weiterhin eine Matrix umfasst, die die Zusammensetzung zu der Stelle des Knochen- oder Knorpeldefekts bringen und eine Struktur zum Induzieren von Knochen- oder Knorpelbildung bereitstellen kann.

18. Pharmazeutische Zusammensetzung nach Anspruch 17, wobei die Matrix Hydroxyapatit, Kollagen, Polymilchsäure oder Tricalciumphosphat umfasst.

19. Verwendung eines Proteins nach Anspruch 14 zur Herstellung einer pharmazeutischen Zusammensetzung zum Induzieren von Knochen- oder Knorpelbildung.

## Revendications

1. Gène encodant la BMP-1 humaine, comprenant la séquence d'ADN suivante :

10 20 30 (1) 50
CTAGAGGCCG CTTCCCTCGC CGCCGCCCCG CCAGC ATG CCC GGC GTG GCC CGC CTG CCG
MET Pro Gly Val Ala Arg Leu Pro

65 80 95 110
CTG CTG CTC GGG CTG CTG CTG CTC CCG CGT CCC GGC CGG CCG CTG GAC TTG GCC
Leu Leu Leu Gly Leu Leu Leu Leu Pro Arg Pro Gly Arg Pro Leu Asp Leu Ala

125 140 155
GAC TAC ACC TAT GAC CTG GCG GAG GAG GAC GAC TCG GAG CCC CTC AAC TAC AAA
Asp Tyr Thr Tyr Asp Leu Ala Glu Glu Asp Asp Ser Glu Pro Leu Asn Tyr Lys

170 185 200 215
GAC CCC TGC AAG GCG GCT GCC TTT CTT GGG GAC ATT GCC CTG GAC GAA GAG GAC
Asp Pro Cys Lys Ala Ala Ala Phe Leu Gly Asp Ile Ala Leu Asp Glu Glu Asp

230 245 260 275
CTG AGG GCC TTC CAG GTA CAG CAG GCT GTG GAT CTC AGA CGG CAC ACA GCT CGT
Leu Arg Ala Phe Gln Val Gln Gln Ala Val Asp Leu Arg Arg His Thr Ala Arg

290 305 320
AAG TCC TCC ATC AAA GCT GCA GTT CCA GGA AAC ACT TCT ACC CCC AGC TGC CAG
Lys Ser Ser Ile Lys Ala Ala Val Pro Gly Asn Thr Ser Thr Pro Ser Cys Gln

335 350 365 380
AGC ACC AAC GGG CAG CCT CAG AGG GGA GCC TGT GGG AGA TGG AGA GGT AGA TCC
Ser Thr Asn Gly Gln Pro Gln Arg Gly Ala Cys Gly Arg Trp Arg Gly Arg Ser

395 410 425
CGT AGC CGG CGG GCG GCG ACG TCC CGA CCA GAG CGT GTG TGG CCC GAT GGG GTC
Arg Ser Arg Arg Ala Ala Thr Ser Arg Pro Glu Arg Val Trp Pro Asp Gly Val

440 455 470 485
ATC CCC TTT GTC ATT GGG GGA AAC TTC ACT GGT AGC CAG AGG GCA GTC TTC CGG
Ile Pro Phe Val Ile Gly Gly Asn Phe Thr Gly Ser Gln Arg Ala Val Phe Arg

500 515 530 545
CAG GCC ATG AGG CAC TGG GAG AAG CAC ACC TGT GTC ACC TTC CTG GAG CGC ACT
Gln Ala MET Arg His Trp Glu Lys His Thr Cys Val Thr Phe Leu Glu Arg Thr

560 575 590
GAC GAG GAC AGC TAT ATT GTG TTC ACC TAT CGA CCT TGC GGG TGC TGC TCC TAC
Asp Glu Asp Ser Tyr Ile Val Phe Thr Tyr Arg Pro Cys Gly Cys Cys Ser Tyr

605 620 635 650
GTG GGT CGC CGC GGC GGG GGC CCC CAG GCC ATC TCC ATC GGC AAG AAC TGT GAC
Val Gly Arg Arg Gly Gly Gly Pro Gln Ala Ile Ser Ile Gly Lys Asn Cys Asp

665 680 695
AAG TTC GGC ATT GTG GTC CAC GAG CTG GGC CAC GTC GTC GGC TTC TGG CAC GAA
Lys Phe Gly Ile Val Val His Glu Leu Gly His Val Val Gly Phe Trp His Glu

710 725 740 755
CAC ACT CGG CCA GAC CGG GAC CGC CAC GTT TCC ATC GTT CGT GAG AAC ATC CAG
His Thr Arg Pro Asp Arg Asp Arg His Val Ser Ile Val Arg Glu Asn Ile Gln

770 785 800 815
CCA GGG CAG GAG TAT AAC TTC CTG AAG ATG GAG CCT CAG GAG GTG GAG TCC CTG
Pro Gly Gln Glu Tyr Asn Phe Leu Lys MET Glu Pro Gln Glu Val Glu Ser Leu

830 845 860
GGG GAG ACC TAT GAC TTC GAC AGC ATC ATG CAT TAC GCT CGG AAC ACA TTC TCC
Gly Glu Thr Tyr Asp Phe Asp Ser Ile MET His Tyr Ala Arg Asn Thr Phe Ser

875 890 905 920
AGG GGC ATC TTC CTG GAT ACC ATT GTC CCC AAG TAT GAG GTG AAC GGG GTG AAA
Arg Gly Ile Phe Leu Asp Thr Ile Val Pro Lys Tyr Glu Val Asn Gly Val Lys

935 950 965
CCT CCC ATT GGC CAA AGG ACA CGG CTC AGC AAG GGG GAC ATT GCC CAA GCC CGC
Pro Pro Ile Gly Gln Arg Thr Arg Leu Ser Lys Gly Asp Ile Ala Gln Ala Arg

980 995 1010 1025
AAG CTT TAC AAG TGC CCA GCC TGT GGA GAG ACC CTG CAA GAC AGC ACA GGC AAC
Lys Leu Tyr Lys Cys Pro Ala Cys Gly Glu Thr Leu Gln Asp Ser Thr Gly Asn

1040 1055 1070 1085
TTC TCC TCC CCT GAA TAC CCC AAT GGC TAC TCT GCT CAC ATG CAC TGC GTG TGG
Phe Ser Ser Pro Glu Tyr Pro Asn Gly Tyr Ser Ala His MET His Cys Val Trp

1100 1115 1130
CGC ATC TCT GTC ACA CCC GGG GAG AAG ATC ATC CTG AAC TTC ACG TCC CTG GAC
Arg Ile Ser Val Thr Pro Gly Glu Lys Ile Ile Leu Asn Phe Thr Ser Leu Asp

1145 1160 1175 1190
CTG TAC CGC AGC CGC CTG TGC TGG TAC GAC TAT GTG GAG GTC CGA GAT GGC TTC
Leu Tyr Arg Ser Arg Leu Cys Trp Tyr Asp Tyr Val Glu Val Arg Asp Gly Phe

1205 1220 1235
TGG AGG AAG GCG CCC CTC CGA GGC CGC TTC TGC GGG TCC AAA CTC CCT GAG CCT
Trp Arg Lys Ala Pro Leu Arg Gly Arg Phe Cys Gly Ser Lys Leu Pro Glu Pro

1250 1265 1280 1295
ATC GTC TCC ACT GAC AGC CGC CTC TGG GTT GAA TTC CGC AGC AGC AGC AAT TGG
Ile Val Ser Thr Asp Ser Arg Leu Trp Val Glu Phe Arg Ser Ser Ser Asn Trp

1310 1325 1340 1355
GTT GGA AAG GGC TTC TTT GCA GTC TAC GAA GCC ATC TGC GGG GGT GAT GTG AAA
Val Gly Lys Gly Phe Phe Ala Val Tyr Glu Ala Ile Cys Gly Gly Asp Val Lys

1370 1385 1400
AAG GAC TAT GGC CAC ATT CAA TCG CCC AAC TAC CCA GAC GAT TAC CGG CCC AGC
Lys Asp Tyr Gly His Ile Gln Ser Pro Asn Tyr Pro Asp Asp Tyr Arg Pro Ser

1415 1430 1445 1460
AAA GTC TGC ATC TGG CGG ATC CAG GTG TCT GAG GGC TTC CAC GTG GGC CTC ACA
Lys Val Cys Ile Trp Arg Ile Gln Val Ser Glu Gly Phe His Val Gly Leu Thr

1475 1490 1505
TTC CAG TCC TTT GAG ATT GAG CGC CAC GAC AGC TGT GCC TAC GAC TAT CTG GAG
Phe Gln Ser Phe Glu Ile Glu Arg His Asp Ser Cys Ala Tyr Asp Tyr Leu Glu

1520 1535 1550 1565
GTG CGC GAC GGG CAC AGT GAG AGC AGC ACC CTC ATC GGG CGC TAC TGT GGC TAT
Val Arg Asp Gly His Ser Glu Ser Ser Thr Leu Ile Gly Arg Tyr Cys Gly Tyr

1580 1595 1610 1625
GAG AAG CCT GAT GAC ATC AAG AGC ACG TCC AGC CGC CTC TGG CTC AAG TTC GTC
Glu Lys Pro Asp Asp Ile Lys Ser Thr Ser Ser Arg Leu Trp Leu Lys Phe Val

1640 1655 1670
TCT GAC GGG TCC ATT AAC AAA GCG GGC TTT GCC GTC AAC TTT TTC AAA GAG GTG
Ser Asp Gly Ser Ile Asn Lys Ala Gly Phe Ala Val Asn Phe Phe Lys Glu Val

1685 1700 1715 1730
GAC GAG TGC TCT CGG CCC AAC CGC GGG GGC TGT GAG CAG CGG TGC CTC AAC ACC
Asp Glu Cys Ser Arg Pro Asn Arg Gly Gly Cys Glu Gln Arg Cys Leu Asn Thr

1745 1760 1775
CTG GGC AGC TAC AAG TGC AGC TGT GAC CCC GGG TAC GAG CTG GCC CCA GAC AAG
Leu Gly Ser Tyr Lys Cys Ser Cys Asp Pro Gly Tyr Glu Leu Ala Pro Asp Lys

1790 1805 1820 1835
CGC CGC TGT GAG GCT GCT TGT GGC GGA TTC CTC ACC AAG CTC AAC GGC TCC ATC
Arg Arg Cys Glu Ala Ala Cys Gly Gly Phe Leu Thr Lys Leu Asn Gly Ser Ile

1850 1865 1880 1895
ACC AGC CCG GGC TGG CCC AAG GAG TAC CCC CCC AAC AAG AAC TGC ATC TGG CAG
Thr Ser Pro Gly Trp Pro Lys Glu Tyr Pro Pro Asn Lys Asn Cys Ile Trp Gln

1910 1925 1940
CTG GTG GCC CCC ACC CAG TAC CGC ATC TCC CTG CAG TTT GAC TTC TTT GAG ACA
Leu Val Ala Pro Thr Gln Tyr Arg Ile Ser Leu Gln Phe Asp Phe Phe Glu Thr

1955 1970 1985 2000
GAG GGC AAT GAT GTG TGC AAG TAC GAC TTC GTG GAG GTG CGC AGT GGA CTC ACA
Glu Gly Asn Asp Val Cys Lys Tyr Asp Phe Val Glu Val Arg Ser Gly Leu Thr

2015 2030 2045
GCT GAC TCC AAG CTG CAT GGC AAG TTC TGT GGT TCT GAG AAG CCC GAG GTC ATC
Ala Asp Ser Lys Leu His Gly Lys Phe Cys Gly Ser Glu Lys Pro Glu Val Ile

2060                2075                2090                2105
ACC TCC CAG TAC AAC AAC ATG CGC GTG GAG TTC AAG TCC GAC AAC ACC GTG TCC
Thr Ser Gln Tyr Asn Asn MET Arg Val Glu Phe Lys Ser Asp Asn Thr Val Ser

        2120                2135                2150                2165
AAA AAG GGC TTC AAG GCC CAC TTC TTC TCA GAA AAG AGG CCA GCT CTG CAG CCC
Lys Lys Gly Phe Lys Ala His Phe Phe Ser Glu Lys Arg Pro Ala Leu Gln Pro

                2180                2195                2210
CCT CGG GGA CGC CCC CAC CAG CTC AAA TTC CGA GTG CAG AAA AGA AAC CGG ACC
Pro Arg Gly Arg Pro His Gln Leu Lys Phe Arg Val Gln Lys Arg Asn Arg Thr
   (730)
  2225       2235       2245       2255       2265       2275       2285
CCC CAG TGAGGCCTGC CAGGCCTCCC GGACCCCTTG TTACTCAGGA ACCTCACCTT GGACGGAATG
Pro Gln .

    2295       2305       2315       2325       2335       2345       2355
GGATGGGGGC TTCGGTGCCC ACCAACCCCC CACCTCCACT CTGCCATTCC GGCCCACCTC CCTCTGGCCG

    2365       2375       2385       2395       2405       2415       2425
GACAGAACTG GTGCTCTCTT CTCCCCACTG TGCCCGTCCG CGGACCGGGG ACCCTTCCCC GTGCCCTACC

    2435       2445       2455       2465       2475       2485       2495
CCCTCCCATT TTGATGGTGT CTGTGACATT TCCTGTTGTG AAGTAAAAGA GGGACCCCTG CGTCCTGCCT

CTAGA

2. Gène encodant la BMP-1 humaine, possédant la séquence d'acides aminés indiquée à la revendication 1.

3. Gène encodant la séquence d'acides aminés de la BMP-1 humaine selon la revendication 1, et comprenant des séquences d'ADN :
(a) qui diffèrent d'une séquence d'ADN selon la revendication 1 dans une séquence de codon à cause de la dégénérescence du code génétique ; ou
(b) qui s'hybrident à une séquence d'ADN selon la revendication 1 ou selon le paragraphe (a) ci-dessus dans des conditions d'hybridation stringentes.

4. Séquence d'ADN selon la revendication 3, à savoir une séquence d'ADN génomique.

5. Séquence d'ADN selon la revendication 3, à savoir une séquence d'ADNc.

6. Gène encodant la BMP-1 bovine, comprenant la séquence d'ADN suivante :

280 290 (1) 308 323
CCTTGCCTCT TCTCTCTCCA GCT GCC TTC CTT GGG GAC ATC GCC CTG GAC GAG GAG
Ala Phe Leu Gly Asp Ile Ala Leu Asp Glu Glu
338 353 368
GAC TTG AGG GCC TTC CAA GTG CAG CAG GCT GCG GAC CTC AGA CAG CGT GCA ACC
Asp Leu Arg Ala Phe Gln Val Gln Gln Ala Ala Asp Leu Arg Gln Arg Ala Thr
383 398 (37) 414 424
CGC AGG TCT TCC ATC AAA GCT GCA GGTACACTGG GTACAGGCCA
Arg Arg Ser Ser Ile Lys Ala Ala

7. Gène encodant la BMP-1 bovine, contenant la séquence d'acides aminés selon la revendication 6.

8. Gène encodant la séquence d'acides aminés de la BMP-1 selon la revendication 6, et comprenant des séquences d'ADN :
(a) qui diffèrent d'une séquence d'ADN selon la revendication 6 dans une séquence de codon à cause de la dégénérescence du code génétique ; ou
(b) qui s'hybrident à une séquence d'ADN selon la revendication 6 ou selon le paragraphe (a) ci-dessus dans des conditions d'hybridation stringentes.

9. Séquence d'ADN selon la revendication 8, à savoir une séquence d'ADN génomique.

10. Séquence d'ADN selon la revendication 8, à savoir une séquence d'ADNc.

11. Vecteur contenant le gène ou la séquence d'ADN selon l'une quelconque des revendications 1 à 10, en association opérante avec une séquence de contrôle de l'expression.

12. Cellule transformée avec un vecteur selon la revendication 11.

13. Cellule selon la revendication 12, à savoir une cellule mammalienne, une cellule bactérienne, une cellule d'insecte ou une cellule de levure.

14. Protéine présentant les propriétés de la BNP-1, qui est encodée par le gène ou par la séquence d'ADN selon l'une quelconque des revendications 1 à 10.

15. Procédé pour la production de la protéine selon la revendication 14, comprenant les étapes de mise en culture, dans un milieu de culture approprié, de la cellule selon la revendication 13, et d'isolation de ladite protéine à partir dudit milieu de culture.

16. Composition pharmaceutique comprenant la protéine selon la revendication 14 et un véhicule pharmaceutiquement acceptable.

17. Composition pharmaceutique selon la revendication 16, comprenant en outre une matrice capable de distribuer la composition au site du défaut osseux ou cartilagineux et de produire une structure destinée à induire une ostéogenèse ou une formation de cartilage.

18. Composition pharmaceutique selon la revendication 17, dans laquelle ladite matrice comprend de l'hydroxyapatite, du collagène, de l'acide polylactique ou du phosphate tricalcique.

19. Utilisation d'une protéine selon la revendication 14, pour la préparation d'une composition pharmaceutique destinée à induire une ostéogenèse ou une formation de cartilage.
